# EUROPÄISCHE PATENTSCHRIFT

(11) **EP 0 752 854 B1**
(45) Veröffentlichungstag und Bekanntmachung des Hinweises auf die Patenterteilung: **22.08.2001**
(21) Anmeldenummer: 95914301.7
(22) Anmeldetag: 23.03.1995
(51) Int. Cl.: A61K 31/00, A61K 31/505, A61K 31/53

(54) **PYRIMIDIN- ODER TRIAZINCARBONSÄUREDERIVATE ZUR VERWENDUNG ALS ARZNEIMITTEL**
PYRIMIDINE OR TRIAZINE CARBOXYLIC ACID DERIVATIVES TO BE USED AS MEDICAMENTS
DERIVES D'ACIDE CARBOXYLIQUE DE PYRIMIDINE OU DE TRIAZINE UTILISES COMME MEDICAMENTS

(30) Priorität: 31.03.1994 DE 4411225
(43) Veröffentlichungstag der Anmeldung: 15.01.1997
(73) Patentinhaber: BASF AKTIENGESELLSCHAFT, 67056 Ludwigshafen (DE)
(72) Erfinder: BAUMANN, Ernst, D-67373 Dudenhofen (DE); VOGELBACHER, Uwe, Josef, D-67071 Ludwigshafen (DE); RHEINHEIMER, Joachim, D-67063 Ludwigshafen (DE); KLINGE, Dagmar, D-69120 Heidelberg (DE); RIECHERS, Hartmut, D-67067 Ludwigshafen (DE); KRÖGER, Burkhard, D-67117 Limburgerhof (DE); BIALOJAN, Siegfried, D-68723 Oftersheim (DE); BOLLSCHWEILER, Claus, D-69118 Heidelberg (DE); WERNET, Wolfgang, D-67454 Hassloch (DE); UNGER, Liliane, D-67065 Ludwigshafen (DE); RASCHACK, Manfred, D-67256 Weisenheim (DE)
(74) Vertreter: Grünecker, Kinkeldey, Stockmair & Schwanhäusser Anwaltssozietät
(86) Internationale Anmeldenummer: EP9501099
(87) Internationale Veröffentlichungsnummer: WO9526716

(56) Entgegenhaltungen:
- EP-A- 0 517 215
- DE-A- 4 313 412

## Beschreibung

Die vorliegende Erfindung betrifft die Verwendung bestimmter Carbonsäurederivate als Arzneimittel.

Endothelin ist ein aus 21 Aminosäuren aufgebautes Peptid, das von vaskulärem Endothel synthetisiert und freigesetzt wird. Endothelin existiert in drei Isoformen, ET-1, ET-2 und ET-3. Im Folgenden bezeichnet "Endothelin" oder "ET" eine oder alle Isoformen von Endothelin. Endothelin ist ein potenter Vasokonstriktor und hat einen starken Effekt auf den Gefäßtonus. Es ist bekannt, daß diese Vasokonstriktion von der Bindung von Endothelin an seinen Rezeptor verursacht wird (Nature, 332, 411-415, 1988; FEBS Letters, 231, 440-444, 1988 und Biochem. Biophys. Res. Commun., 154, 868-875, 1988).

Erhöhte oder abnormale Freisetzung von Endothelin verursacht eine anhaltende Gefäßkontraktion in peripheren, renalen und zerebralen Blutgefäßen, die zu Krankheiten führen kann. Wie in der Literatur berichtet, wurden erhöhte Plasmaspiegel von Endothelin gefunden bei Patienten mit Hypertonie, akutem Myokardinfarkt, pulmonärer Hypertonie, Raynaud-Syndrom, Atherosklerose und in den Atemwegen von Asthmatikern (Japan J. Hypertension, 12, 79 (1989), J. Vascular Med. Biology 2, 207 (1990), J. Am. Med. Association 264, 2868 (1990)).

Demnach sollten Substanzen, die spezifisch die Bindung von Endothelin an den Rezeptor inhibieren, auch die obengenannten verschiedenen physiologischen Effekte von Endothelin antagonisieren und daher wertvolle Pharmaka darstellen.

Es wurde nun gefunden, daß bestimmte Carbonsäurederivate gute Hemmstoffe für Endothelinrezeptoren sind.

Gegenstand der Erfindung ist die Verwendung von Carbonsäurederivaten mit der im folgenden beschriebenen Formel I zur Herstellung von Arzneimitteln, insbesondere zur Herstellung von Hemmstoffen für Endothelinrezeptoren.

Carbonsäurederivate der allgemeinen Formel I 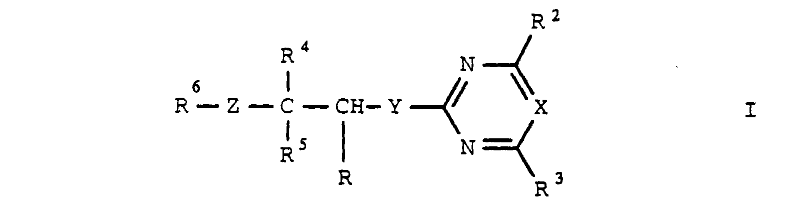 in der R eine Formylgruppe, eine Gruppe CO₂H oder einen zu COOH hydrolysierbaren Rest bedeutet und die übrigen Substituenten folgende Bedeutung haben:
- R²: Halogen, C₁-C₄-Alkyl, C₁-C₄-Halogenalkyl, C₁-C₄-Alkoxy, C₁-C₄-Halogenalkoxy oder C₁-C₄-Alkylthio;
- X: Stickstoff oder CR¹⁴, wobei R¹⁴ Wasserstoff bedeutet oder zusammen mit R³ eine 3- bis 4-gliedrige Alkylen- oder Alkenylenkette bildet, in der jeweils eine Methylengruppe durch Sauerstoff ersetzt ist;
- R³: Halogen, C₁-C₄-Alkyl, C₁-C₄-Halogenalkyl, C₁-C₄-Alkoxy, C₁-C₄-Halogenalkoxy, C₁-C₄-Alkylthio oder R³ ist mit R¹⁴ wie oben angegeben zu einem 5- oder 6-gliedrigen Ring verknüpft;
- R⁴: eine C₁-C₁₀-Alkylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₁-C₁₀-Alkylgruppe, welche ein bis fünf Halogenatome tragen kann und einen der folgenden Reste trägt: ein fünfgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio und/oder Phenyl;
eine C₃-C₁₂-Cycloalkyl- oder C₃-C₁₂-Cycloalkenylgruppe, die ein Sauerstoff- oder Schwefelatom enthalten kann und ein bis fünf Halogenatome und/oder einen der folgenden Reste tragen kann: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₃-C₆-Alkenyl- oder eine C₃-C₆-Alkinylgruppe, welche jeweils ein bis fünf Halogenatome und/oder einen der folgenden Reste tragen kann: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
ein fünf- oder sechsgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
Phenyl oder Naphthyl, die durch einen oder mehrere der folgenden Reste substituiert sein können: Halogen, Nitro, Cyano, Hydroxy, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, C₁-C₄-Alkylamino oder C₁-C₄-Dialkylamino;
R⁴ und R⁵ bilden zusammen mit dem benachbarten Kohlenstoffatom einen 3- bis 8-gliedrigen Ring, der ein Sauerstoff- oder Schwefelatom enthalten kann und einen bis drei der folgenden Reste tragen kann: C₁-C₄-Alkyl, Halogen, C₁-C₄-Halogenalkyl, C₁-C₄-Alkoxy, C₁-C₄-Halogenalkoxy und/oder C₁-C₄-Akylthio;
- R⁵: Wasserstoff, C₁-C₄-Alkyl, C₃-C₆-Alkenyl, C₃-C₆-Alkinyl, C₃-C₈-Cycloalkyl, C₁-C₄-Halogenalkyl, C₁-C₄-Alkoxyalkyl, C₁-C₄-Alkylthioalkyl, Phenyl oder R⁵ ist mit R⁴ wie oben angegeben zu einem 3- bis 8-gliedrigen Ring verknüpft;
- R⁶: C₁-C₈-Alkyl, C₃-C₆-Alkenyl, C₃-C₆-Alkinyl oder C₃-C₈-Cycloalkyl, wobei diese Reste jeweils ein- oder mehrfach substituiert sein können durch: Halogen, Nitro, Cyano, C₁-C₄-Alkoxy, C₃-C₆-Alkenyloxy, C₃-C₆-Alkinyloxy, C₁-C₄-Alkylthio, C₁-C₄-Halogenalkoxy, C₁-C₄-Alkylcarbonyl, C₁-C₄-Alkoxycarbonyl, C₁-C₄-Alkylamino, Di-C₁-C₄-alkylamino, Phenyl, ein- oder mehrfach, z.B. ein bis dreifach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl oder Phenoxy;
Phenyl oder Naphthyl, die jeweils durch einen oder mehrere der folgenden Reste substituiert sein können: Halogen, Nitro, Cyano, Hydroxy, Amino, C₁-C₄-Alkyl, C₁-C₄-Halogenalkyl, C₁-C₄-Alkoxy, C₁-C₄-Halogenalkoxy, Phenoxy, C₁-C₄-Alkylthio, C₁-C₄-Alkylamino oder C₁-C₄-Dialkylamino;
ein fünf- oder sechsgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
- Y: Schwefel oder Sauerstoff oder eine Einfachbindung;
- z: Schwefel oder Sauerstoff;

Die Herstellung der erfindungsgemäßen Verbindungen geht aus von den Epoxiden IV, die man in allgemein bekannter Weise, z.B. wie in J. March, Advanced Organic Chemistry, 2nd ed., 1983, S. 862 und S. 750 beschrieben, aus den Aldehyden bzw. Ketonen II oder den Olefinen III erhält: 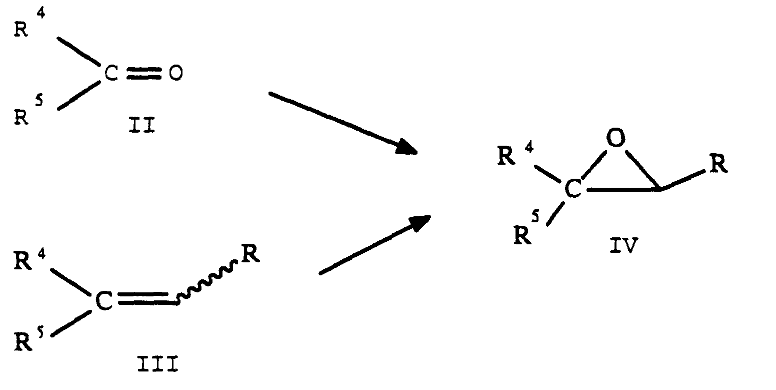 Carbonsäurederivate der allgemeinen Formel VI können hergestellt werden, indem man die Epoxide der allgemeinen Formel IV (z.B. mit R = COOR¹⁰) mit Alkoholen oder Thiolen der allgemeinen Formel V, in der R⁶ und Z die in Anspruch 1 genannte Bedeutung haben, zur Reaktion bringt.

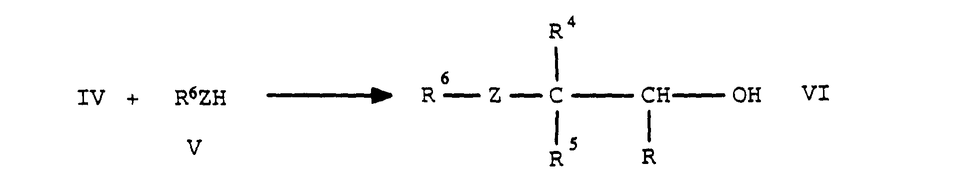

Dazu werden Verbindungen der allgemeinen Formel IV mit einem Überschuß der Verbindungen der Formel V, z.B. 1,2-7, bevorzugt 2-5 Moläquivalenten, auf eine Temperatur von 50 - 200°C, bevorzugt 80 - 150°C, erhitzt.

Die Reaktion kann auch in Gegenwart eines Verdünnungsmittels erfolgen. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide und Sulfone, wie zum Beispiel Dimethylsulfoxid und Sulfolan, und Basen, wie zum Beispiel Pyridin.

Die Reaktion wird dabei bevorzugt in einem Temperaturbereich zwischen 0°C und dem Siedepunkt des Lösungsmittels bzw. Lösungsmittelgemisches durchgeführt.

Die Gegenwart eines Reaktionskatalysators kann von Vorteil sein. Als Katalysatoren kommen dabei starke organische und anorganische Säuren sowie Lewissäuren in Frage. Beispiele hierfür sind unter anderem Schwefelsäure, Salzsäure, Trifluoressigsäure, Bortrifluorid-Etherat und Titan(IV)-Alkoholate.

Die erfindungsgemäßen Verbindungen, in denen Y Sauerstoff bedeutet und die restlichen Substituenten die unter der allgemeinen Formel I angegebenen Bedeutung haben, können beispielsweise derart hergestellt werden, daß man die Carbonsäurederivate der allgemeinen Formel VI, in denen die Substituent en die angegebene Bedeutung haben, mit Verbindungen der allgemeinen Formel VII, 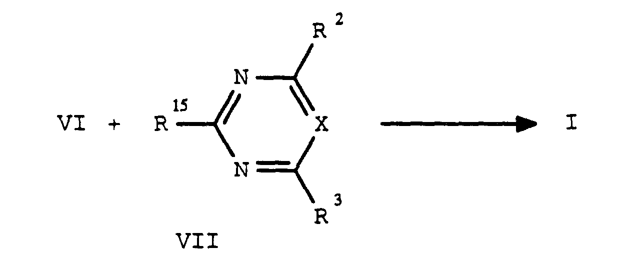 in der R¹⁵ Halogen oder R¹⁶-SO₂- bedeutet, wobei R¹⁶ C₁-C₄-Alkyl, C₁-C₄-Halogenalkyl oder Phenyl sein kann, zur Reaktion bringt. Die Reaktion findet bevorzugt in einem der oben genannten inerten Verdünnungsmittel unter Zusatz einer geeigneten Base, d.h. einer Base, die eine Deprotonierung des Zwischenproduktes VI bewirkt, in einem Temperaturbereich von Raumtemperatur bis zum Siedepunkt des Lösungsmittels statt.

Als Base kann ein Alkali- oder Erdalkalimetallhydrid wie Natriumhydrid, Kaliumhydrid oder Calciumhydrid, ein Carbonat wie Alkalimetallcarbonat, z.B. Natrium- oder Kaliumcarbonat, ein Alkali- oder Erdalkalimetallhydroxid wie Natrium- oder Kaliumhydroxid, eine metallorganische Verbindung wie Butyllithium oder ein Alkaliamid wie Lithiumdiisopropylamid dienen.

Die erfindungsgemäßen Verbindungen, in denen Y Schwefel bedeutet und die restlichen Substituenten die unter der allgemeinen Formel I angegebene Bedeutung haben, können beispielsweise derart hergestellt werden, daß man Carbonsäurederivate der allgemeinen Formel VIII, die in bekannter Weise aus Verbindungen der allgemeinen Formel VI erhältlich sind und in denen die Substituenten die oben angegebene Bedeutung haben, mit Verbindungen der allgemeinen Formel IX, in der R², R³ und X die unter der allgemeinen Formel I angegebene Bedeutung haben, zur Reaktion bringt.

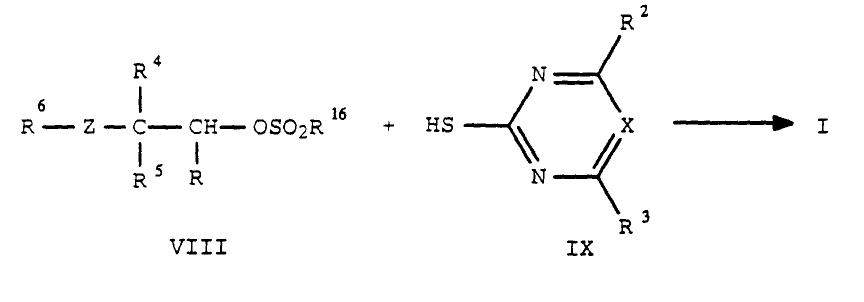

Die Reaktion findet bevorzugt in einem der oben genannten inerten Verdünnungsmittel unter Zusatz einer geeigneten Base, d.h. eine Base, die eine Deprotonierung des Zwischenproduktes IX bewirkt, in einem Temperaturbereich von Raumtemperatur bis zum Siedepunkt des Lösungsmittels statt.

Als Base können neben den oben genannten auch organische Basen wie tertiäre Amine, z.B. Triethylamin, Pyridin, Imidazol oder Diazabicycloundecen dienen.

Verbindungen der Formel I können auch dadurch hergestellt werden, daß man von den entsprechenden Carbonsäuren, d. h. Verbindungen der Formel I, in denen R¹ Hydroxyl bedeutet, ausgeht und diese zunächst auf übliche Weise in eine aktivierte Form wie ein Halogenid, ein Anhydrid oder Imidazolid überführt und dieses dann mit einer entsprechenden Hydroxylverbindung HOR¹⁰ umsetzt. Diese Umsetzung läßt sich in den üblichen Lösungsmitteln durchführen und erfordert oft die Zugabe einer Base, wobei die oben genannten in Betracht kommen. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Außerdem können Verbindungen der Formel I auch dadurch hergestellt werden, daß man von den Salzen der entsprechenden Carbonsäuren ausgeht, d. h. von Verbindungen der Formel I, in denen R für eine Gruppe COR¹ und R¹ für OM stehen, wobei M ein Alkalimetallkation oder das Äquivalent eines Erdalkalimetallkations sein kann. Diese Salze lassen sich mit vielen Verbindungen der Formel R¹-A zur Reaktion bringen, wobei A eine übliche nucleofuge Abgangsgruppe bedeutet, beispielsweise Halogen wie Chlor, Brom, Iod oder gegebenenfalls durch Halogen, Alkyl oder Halogenalkyl substituiertes Aryl- oder Alkylsulfonyl wie z.B. Toluolsulfonyl und Methylsulfonyl oder eine andere äquivalente Abgangsgruppe. Verbindungen der Formel R¹-A mit einem reaktionsfähigen Substituenten A sind bekannt oder mit dem allgemeinen Fachwissen leicht zu erhalten. Diese Umsetzung läßt sich in den üblichen Lösungsmitteln durchführen und wird vorteilhaft unter Zugabe einer Base, wobei die oben genannten in Betracht kommen, vorgenommen.

Der Rest R in Formel I ist breit variabel. Beispielsweise steht R für eine Gruppe 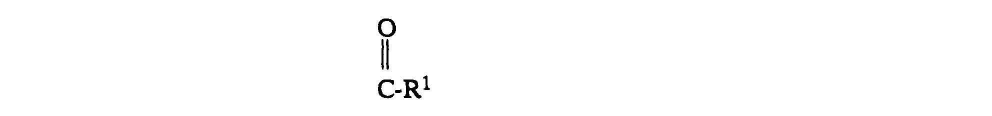 in der R¹ die folgende Bedeutung hat:
a) Wasserstoff;
b) eine Succinimidyloxygruppe;
c) ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder ein bis zwei der folgenden Reste tragen kann:
   C₁-C₄-Alkyl wie Methyl, Ethyl, 1-Propyl, 2-Propyl, 2-Methyl-2-propyl, 2-Methyl-1-propyl, 1-Butyl, 2-Butyl;
   C₁-C₄-Halogenalkyl, insbesondere C₁-C₂-Halogenalkyl wie beispielsweise Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
   C₁-C₄-Halogenalkoxy, insbesondere C₁-C₂-Halogenalkoxy wie Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
   C₁-C₄-Alkoxy wie Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
   C₁-C₄-Alkylthio wie Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
d) 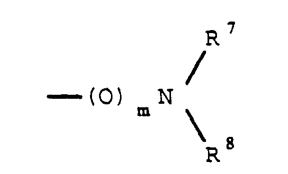 in dem m für 0 oder 1 steht und R⁷ und R⁸, die gleich oder unterschiedlich sein können, die folgende Bedeutung haben:
   Wasserstoff
   C₁-C₈-Alkyl, insbesondere C₁-C₄-Alkyl wie oben genannt;
   C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-l-methyl-2-propenyl und 1-Ethyl-2-methyl-2-propenyl, insbesondere 2-Propenyl, 2-Butenyl, 3-Methyl-2-butenyl und 3-Methyl-2-pentenyl;
   C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl
   C₃-C₈-Cycloalkyl, wie Cyclopropyl, Cyclobutyl, Cyclopentyl, Cyclohexyl, Cycloheptyl und Cyclooctyl, wobei diese Alkyl-, Cycloalkyl-, Alkenyl- und Alkinylgruppen jeweils ein bis fünf Halogenatome, insbesondere Fluor oder Chlor und/oder ein bis zwei der folgenden Gruppen tragen können:
   C₁-C₄-Alkyl, C₁-C₄-Alkoxy, C₁-C₄-Alkylthio, C₁-C₄-Halogenalkoxy wie vorstehend genannt, C₃-C₆-Alkenyloxy, C₃-C₆-Alkenylthio, C₃-C₆-Alkinyloxy, C₃-C₆-Alkinylthio, wobei die in diesen Resten vorliegenden Alkenyl- und Alkinylbestandteile vorzugsweise den oben genannten Bedeutungen entsprechen;
   C₁-C₄-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl;
   C₁-C₄-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl;
   C₃-C₆-Alkenylcarbonyl, C₃-C₆-Alkinylcarbonyl, C₃-C₆-Alkenyloxycarbonyl und C₃-C₆-Alkinyloxycarbonyl, wobei die Alkenyl- bzw. Alkinylreste vorzugsweise, wie voranstehend im einzelnen aufgeführt, definiert sind;
   Phenyl, gegebenenfalls ein- oder mehrfach, z.B. ein- bis dreifach substituiert durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio wie beispielsweise 2-Fluorphenyl, 3-Chlorphenyl, 4-Bromphenyl, 2-Methylphenyl, 3-Nitrophenyl, 4-Cyanophenyl, 2-Trifluormethylphenyl, 3-Methoxyphenyl, 4-Trifluorethoxyphenyl, 2-Methylthiophenyl, 2,4-Dichlorphenyl, 2-Methoxy-3-methylphenyl, 2,4-Dimethoxyphenyl, 2-Nitro-5-cyanophenyl, 2,6-Difluorphenyl;
   Di-C₁-C₄-Alkylamino wie insbesondere Dimethylamino, Dipropylamino, N-Propyl-N-methylamino, N-Propyl-N-ethylamino, Diisopropylamino, N-Isopropyl-N-methylamino, N-Isopropyl-N-ethylamino, N-Isopropyl-N-propylamino;
   R⁷ und R⁸ ferner Phenyl, das durch einen oder mehrere, z.B. ein bis drei der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio, wie insbesondere oben genannt;
   oder R⁷ und R⁸ bilden gemeinsam eine zu einem Ring geschlossene, optionell substituierte, z.B. durch C₁-C₄-Alkyl substituierte C₄-C₇-Alkylenkette, die ein Heteroatom, ausgewählt aus der Gruppe Sauerstoff, Schwefel oder Stickstoff, enthalten kann wie -(CH₂)₄-, -(CH₂)₅-, -(CH₂)₆-, -(CH₂)₇-, -(CH₂)₂-O-(CH₂)₂-, -CH₂-S-(CH₂)₃-, -(CH₂)₂-O-(CH₂)₃-, -NH-(CH₂)₃-, -CH₂-NH-(CH₂)₂-, -CH₂-CH=CH-CH₂-, -CH=CH-(CH₂)₃-;
e) 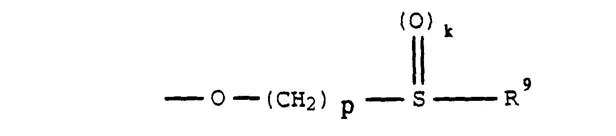 in der k die Werte 0, 1 und 2, p die Werte 1, 2, 3 und 4 annehmen und R⁹ für
   C₁-C₄-Alkyl, C₁-C₄-Halogenalkyl, C₃-C₆-Alkenyl, C₃-C₆-Alkinyl oder gegebenenfalls substituiertes Phenyl steht, wie insbesondere oben genannt.
f) R¹ ferner ein Rest OR¹⁰, worin R¹⁰ bedeutet:
   Wasserstoff, das Kation eines Alkalimetalls wie Lithium, Natrium, Kalium oder das Kation eines Erdalkalimetalls wie Calcium, Magnesium und Barium oder ein umweltverträgliches organisches Ammoniumion wie tertiäres C₁-C₄-Alkylammonium oder das Ammoniumion;
   C₃-C₈-Cycloalkyl wie vorstehend genannt, welches ein bis drei C₁-C₄-Alkylgruppen tragen kann;
   C₁-C₈-Alkyl wie insbesonder Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, welches ein bis fünf Halogenatome, insbesondere Fluor und Chlor und/oder einen der folgenden Reste tragen kann:
   C₁-C₄-Alkoxy, C₁-C₄-Alkylthio, Cyano, C₁-C₄-Alkylcarbonyl, C₃-C₈-Cycloakyl, C₁-C₄-Alkoxycarbonyl, Phenyl, Phenoxy oder Phenylcarbonyl, wobei die aromatischen Reste ihrerseits jeweils ein bis fünf Halogenatome und/oder ein bis drei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
   eine C₁-C₈-Alkylgruppe wie vorstehend genannt, welch ein bis fünf Halogenatome, insbesonder Fluor und/oder Chlor tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff- oder Schwefelatom, welcher ein bis vier Halogenatome und/oder ein bis zwei der folgenden Reste tragen kann:
   Nitro, Cyano, C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-l-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl;
   eine C₂-C₆-Alkylgrupe, welche in der 2-Position einen der folgenden Reste trägt: C₁-C₄-Alkoxyimino, C₃-C₆-Alkinyloxyimino, C₃-C₆-Halogenalkenyloxyimino oder Benzyloxyimino;
   eine C₃-C₆-Alkenyl- oder eine C₃-C₆-Alkinylgruppe, wobei diese Gruppen ihrerseits ein bis fünf Halogenatome tragen können;
   R¹⁰ ferner ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
   ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-l-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-1-yl;
   R¹⁰ ferner ein Gruppe 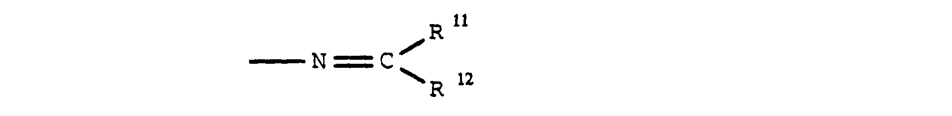 worin R¹¹ und R¹², die gleich oder verschieden sein können, bedeuten:
   C₁-C₈-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio und/oder einen gegebenenfalls substituierten Phenylrest, wie insbesondere vorstehend genannt, tragen können;
   Phenyl, das durch einen oder mehrere, z.B. einen bis drei der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio, wobei diese Reste insbesondere den oben genannten entsprechen;
   oder R¹¹ und R¹² bilden gemeinsam eine C₃-C₁₂-Alkylenkette, welche ein bis drei C₁-C₄-Alkylgruppen tragen und ein Heteroatom aus der Gruppe Sauerstoff, Schwefel und Stickstoff enthalten kann, wie insbesondere bei R⁷ und R⁸ genannt.
g) R¹ ferner ein Rest 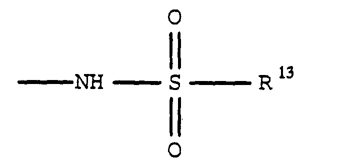 worin R¹³ bedeutet:
   C₁-C₄-Alkyl, C₃-C₆-Alkenyl, C₃-C₆-Alkinyl, C₃-C₈-Cycloalkyl wie insbesondere vorstehend genannt, wobei diese Reste einen C₁-C₄-Alkoxy-, C₁-C₄-Alkylthio- und/oder einen Phenylrest wie oben genannt tragen können;
   Phenyl, gegebenenfalls substituiert, insbesondere wie vorstehend genannt.

Im Hinblick auf die biologische Wirkung sind Carbonsäurederivate der allgemeinen Formel I bevorzugt, in denen die Substituenten folgende Bedeutung haben:
- R²: die bei R¹ im einzelnen genannten C₁-C₄-Alkyl-, C₁-C₄-Halogenalkyl-, C₁-C₄-Alkoxy-, C₁-C₄-Halogenalkoxy-, C₁-C₄-Alkylthiogruppen und Halogenatome, insbesondere Chlor, Methyl, Methoxy, Ethoxy, Difluormethoxy, Trifluormethoxy, besonders bevorzugt Methoxy;
- X: Stickstoff oder CR¹⁴, worin
- R¹⁴: Wasserstoff bedeutet oder zusammen mit R³ eine 4- bis 5-gliedrige Alkylen- oder Alkenylenkette bildet, in der jeweils eine Methylengruppe durch Sauerstoff ersetzt ist wie -CH₂-CH₂-O-, -CH=CH-O-, -CH₂-CH₂-CH₂-O-, -CH=CH-CH₂O-, insbesondere Wasserstoff und -CH₂-CH₂-O-;
- R³: die bei R¹ genannten C₁-C₄-Alkyl-, C₁-C₄-Halogenalkyl-, C₁-C₄-Alkoxy-, C₁-C₄-Halogenalkoxy-, C₁-C₄-Alkylthiogruppen und Halogenatome, insbesondere Chlor, Methyl, Methoxy, Ethoxy, Difluormethoxy, Trifluormethoxy oder mit R¹⁴ wie oben genannt zu einem 5- oder 6-gliedrigen Ring verknüpft ist, besonders bevorzugt steht R³ für Methoxy;
- R⁴: C₁-C₁₀-Alkyl wie bei R¹ im einzelnen genannt, welches ein bis fünf Halogenatome wie Fluor, Chlor, Brom, Jod, insbesondere Fluor und Chlor und/oder einen der folgenden Reste tragen kann: Alkoxy, Alkylthio, Cyano, Alkylcarbonyl, Alkoxycarbonyl, Phenyl, Phenoxy, Phenylcarbonyl wie im allgemeinen und besonderen bei R¹ genannt;
C₁-C₁₀-Alkyl wie vorstehend genannt, welches ein bis fünf Halogenatome wie vorstehend genannt, insbesondere Fluor und Chlor, tragen kann und einen ggf. substituierten 5-gliedrigen Heteroaromaten, wie voranstehend für R¹ genannt, trägt;
C₃-C₁₂-Cycloalkyl, insbesondere C₃-C₇-Cycloalkyl oder C₃-C₁₂-Cycloalkenyl, insbesondee C₄-C₇-Cycloalkenyl, wobei im gesättigten oder ungesättigten Ring eine Methylengruppe durch ein Sauerstoff- oder Schwefelatom ersetzt sein kann, wie Cyclopropyl, Cyclobutyl, Cyclopentyl, Cyclohexyl, Cycloheptyl, Tetrahydrofuranyl, Tetrahydrothienyl, Tetrahydropyranyl, Tetrahydrothiopyranyl, Cyclopropenyl, Dihydrofuranyl, Dihydrothienyl, Dihydropyranyl, Dihydrothiopyranyl, wobei die Cycloalkyl- bzw. Cycloalkenylreste substituiert sein können durch ein bis fünf Halogenatome wie vorstehend genannt, insbesondere Fluor oder Chlor und/oder einen der folgende Reste: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy, Phenylcarbonyl wie im allgemeinen und besonderen oben genannt;
C₃-C₆-Alkenyl oder C₃-C₆-Alkinyl wie bei R¹ genannt, welche ein bis fünf Halogenatome wie vorstehend genannt, insbesondere Fluor und Chlor und/oder einen der folgenden Reste tragen können:
C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy, Phenylcarbonyl wie im allgemeinen und besonderen oben genannt;
ein 5- oder 6-gliedriges Heteroaryl wie Furyl, Thienyl, Pyrryl, Pyrazolyl, Imidazolyl, Triazolyl, Isoxazolyl, Oxazolyl, Isothiazolyl, Thiazolyl, Thiadiazolyl, Pyridyl, Pyrimidinyl, Pyrazinyl, Pyridazinyl, Triazinyl, beispielsweise 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrazolyl, 4-Pyrazolyl, 5-Pyrazolyl, 2-Pyridyl, 3-Pyridyl, 4-Pyridyl, Oxa-2,4-diazolyl, Oxa-3,4-diazoylyl, Thia-2,4-diazolyl, Thia-3,4-diazolyl und Triazolyl, wobei die Heteroaromaten ein bis fünf Halogenatome wie vorstehend genannt, insbesondere Fluor und Chlor und/oder einen bis drei der folgenden Reste tragen können:
C₁-C₄-Alkyl, C₁-C₄-Alkoxy, C₁-C₄-Alkylthio, Cyano, Nitro, C₁-C₈-Alkylcarbonyl, C₁-C₈-Alkoxycarbonyl, Phenyl, Phenoxy, Phenylcarbonyl wie im allgemeinen und besonderen oben genannt;
R⁴ ferner Phenyl oder Naphthyl, die durch einen oder mehrere, z.B. einen bis drei der folgenden Reste substituiert sein können: Halogen, Nitro, Cyano, Hydroxy, Mercapto, Amino, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino, Di-C₁-C₄-alkylamino, C₁-C₄-Alkylcarbonyl, C₁-C₄-Alkoxycarbonyl, insbesondere wie bei R⁷ und R⁸ genannt, sowie beispielsweise 3-Hydroxyphenyl, 4-Dimethylaminophenyl, 2-Mercaptophenyl, 3-Methoxycarbonylphenyl, 4-Acetylphenyl, 1-Naphthyl, 2-Naphthyl, 3-Brom-2-naphthyl, 4-Methyl-1-naphthyl, 5-Methoxy-1-naphthyl 6-Trifluormethyl-l-naphthyl, 7-Chlor-1-naphthyl, 8-Hydroxy-1-naphthyl;
oder R⁴ bildet mit R⁵ zusammen mit dem benachbarten Kohlenstoffatom einen 3- bis 6-gliedrigen Ring, der ein Sauerstoff- oder Schwefelatom enthalten kann und unsubstituiert ist oder je nach Ringgröße einen bis drei der folgenden Reste trägt: C₁-C₄-Alkyl, C₁-C₄-Alkoxy, C₁-C₄-Halogenalkyl, C₁-C₄-Halogenalkoxy, C₁-C₄-Alkylthio wie im allgemeinen und besonderen oben genannt;
- R⁵: Wasserstoff, C₁-C₄-Alkyl, C₃-C₆-Alkenyl, C₃-C₆-Alkinyl, C₃-C₈-Cycloalkyl, C₁-C₄-Halogenalkyl, C₁-C₄-Alkoxyalkyl, C₁-C₄-Alkylthioalkyl oder Phenyl wie insbesondere vorstehend bei R⁴ genannt;
- R⁶: C₁-C₈-Alkyl, C₃-C₆-Alkenyl, C₃-C₆-Alkinyl oder C₃-C₈-Cycloalkyl wie insbesondere oben genannt, wobei diese Reste jeweils ein-oder mehrfach substituiert sein können durch: Halogen, Nitro, Cyano, C₁-C₄-Alkoxy, C₃-C₆-Alkenyloxy, C₃-C₆-Alkinyloxy, C₁-C₄-Alkylthio, C₁-C₄-Halogenalkoxy, C₁-C₄-Alkylcarbonyl, C₁-C₄-Alkoxycarbonyl, C₁-C₄-Alkylamino, Di-C₁-C₄-alkylamino oder gegebenenfalls substituiertes Phenyl oder Phenoxy, wie insbesondere vorstehend genannt;
Phenyl oder Naphthyl, das durch einen oder mehreren der folgenden Reste substituiert sein kann: Halogen, Nitro, Cyano, Hydroxy, Amino, C₁-C₄-Alkyl, C₁-C₄-Halogenalkyl, C₁-C₄-Alkoxy, C₁-C₄-Halogenalkoxy, Phenoxy, C₁-C₄-Alkylthio, C₁-C₄-Akylamino oder C₁-C₄-Dialkylamino, wie insbesondere bei R⁷ und R⁴ genannt;
ein fünf- oder sechsgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio, wie insbesondere bei R⁴ genannt;
- Y: Schwefel, Sauerstoff oder eine Einfachbindung
- Z: Schwefel oder Sauerstoff.

Besonders bevorzugt sind Verbindungen der Formel I, in der R² und R³ Methoxy und X CH bedeuten. Weiterhin bevorzugt sind Verbindungen der Formel I, in der R² und R³ Methoxy, X CH, Y und Z Sauerstoff und R⁵ C₁-C₄-Alkyl bedeuten. Bevorzugter Rest im Fall von R¹ ist die Gruppe OR¹⁰, wobei R¹⁰ Wasserstoff oder C₁-C₄-Alkyl bedeutet.

R⁴ steht besonders bevorzugt für C₁-C₄-Alkyl, gegebenenfalls substituiertes Phenyl oder einen aromatischen heterocyclischen Rest enthaltend ein Heteroatom wie Furyl oder Thienyl.

R⁶ steht besonders bevorzugt für Phenyl, ggf. 1 - 3fach substituiert durch Halogen, C₁-C₄-Alkyl, C₁-C₄-Alkoxy und/oder C₁-C₄-Alkylthio.

Beispiele für bevorzugte Verbindungen sind in der nachfolgenden Tabelle aufgeführt.

Besonders bevorzugt werden die Verbindungen 4.42 und 4.58 (Beispiel 10, Tab. 4) für die erfindungsgemäße Verwendung eingesetzt.

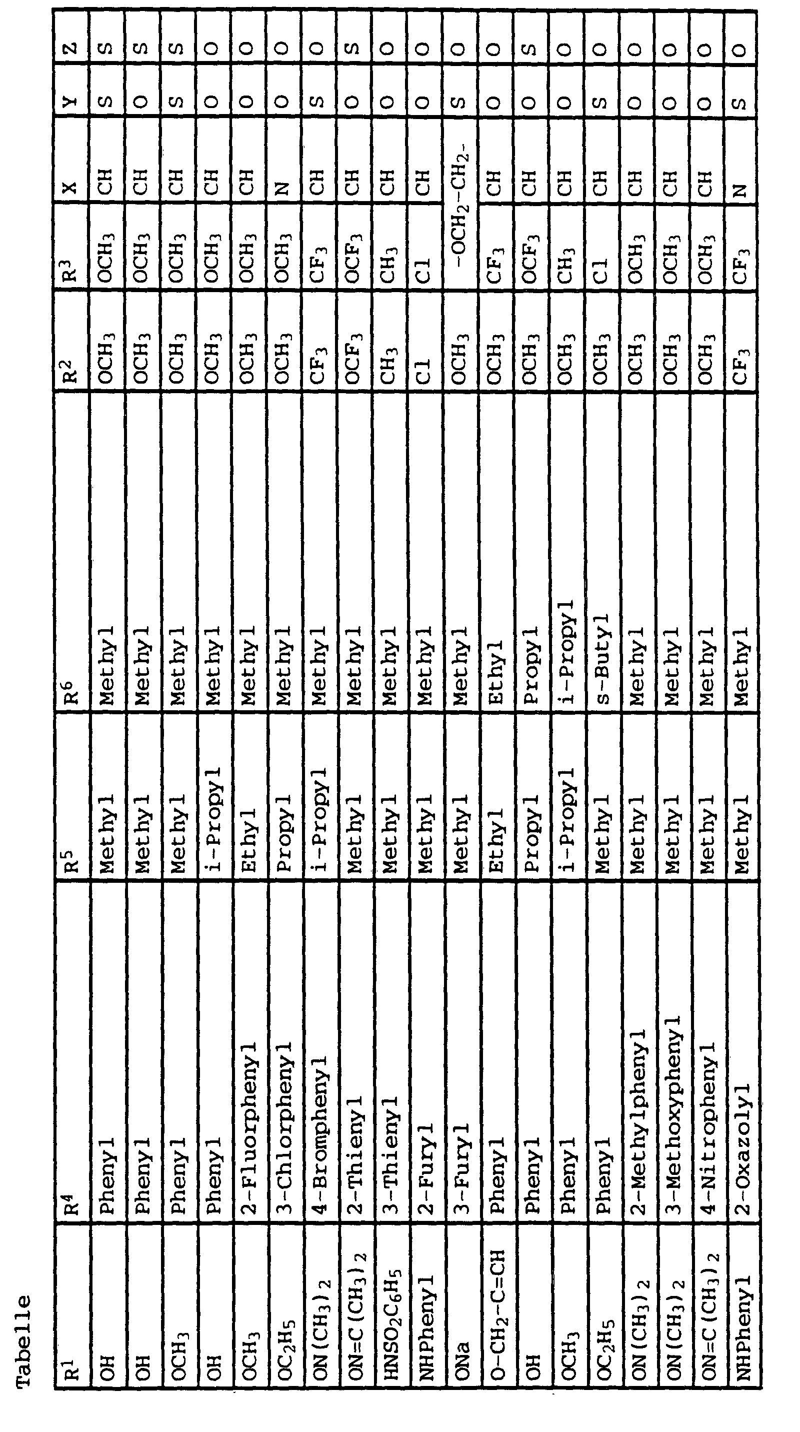

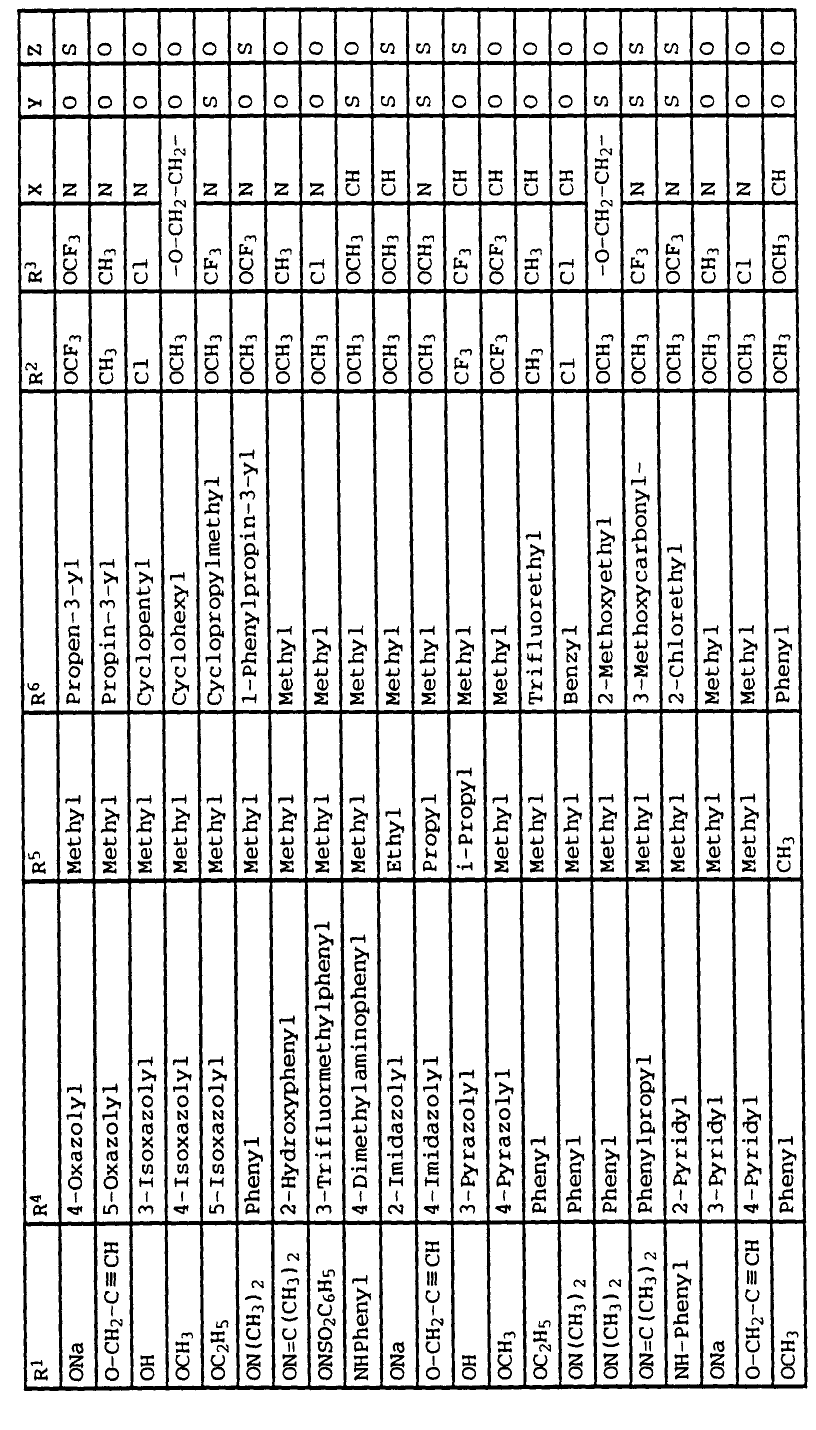

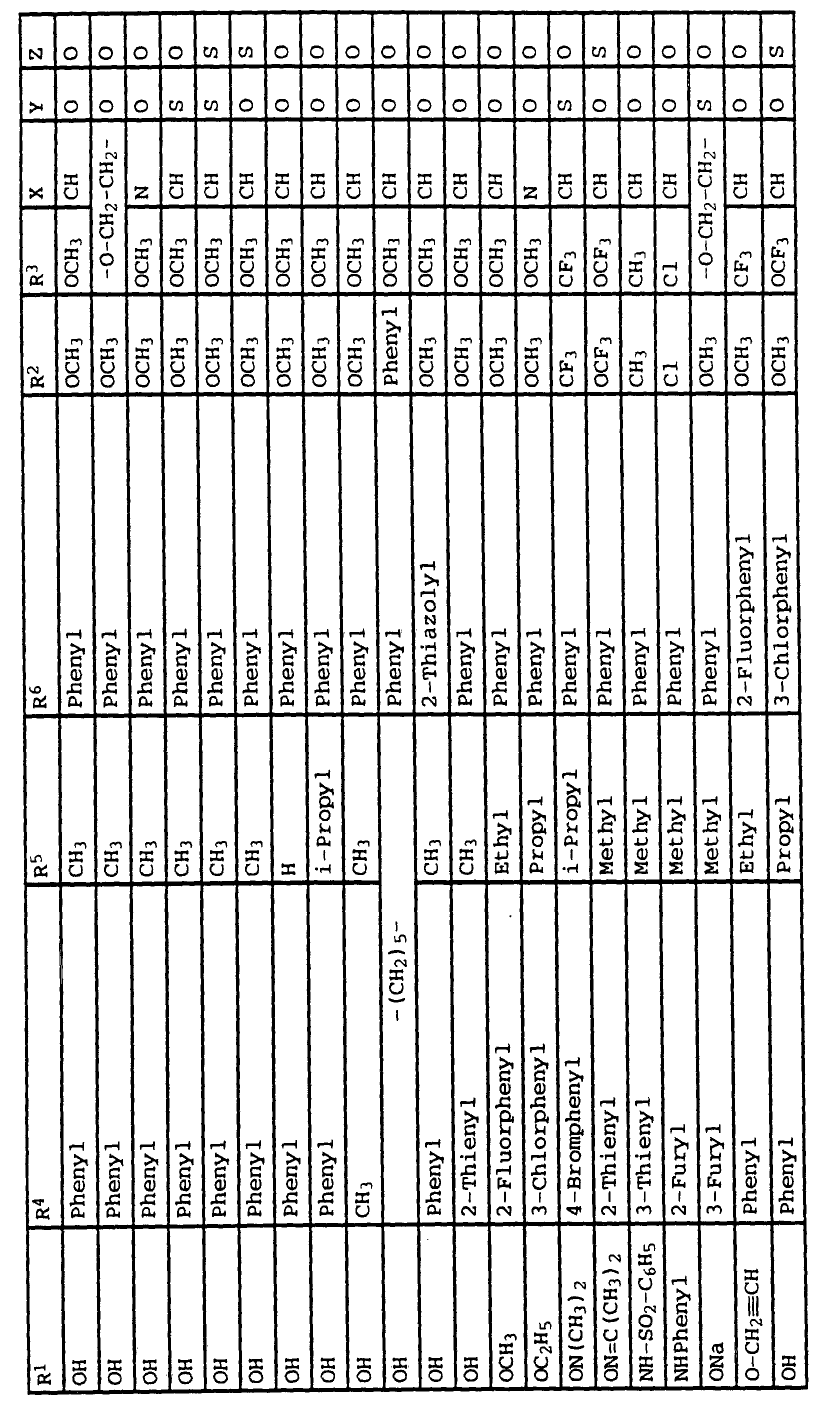

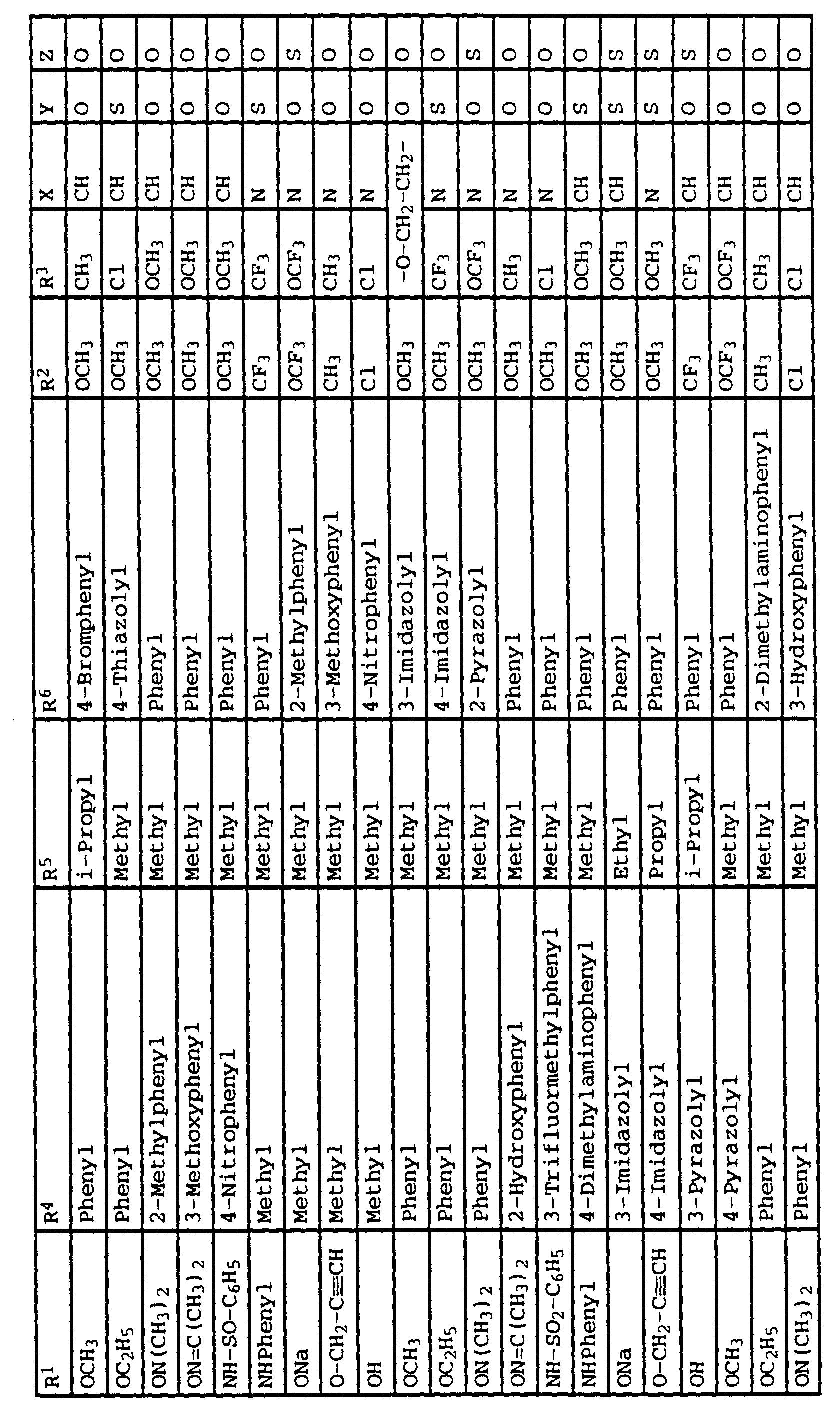

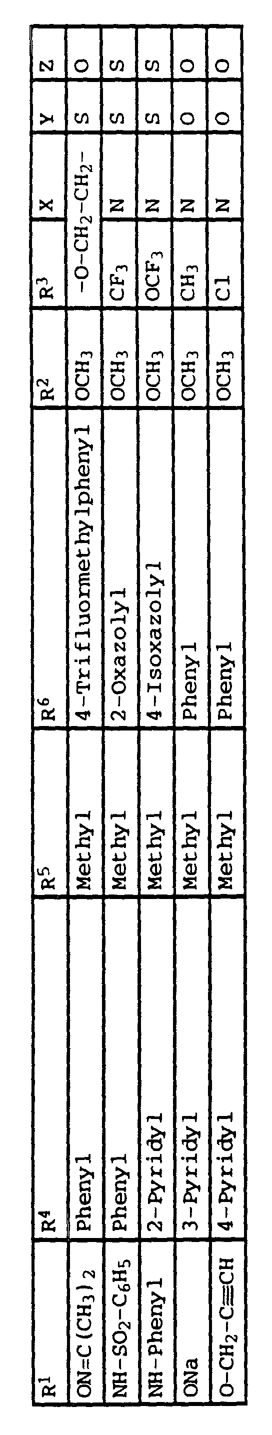

Die Verbindungen der vorliegenden Erfindung bieten ein neues therapeutisches Potential für die Behandlung von Hypertonie, pulmonalem Hochdruck, Myokardinfarkt, Angina Pectoris, akutem Nierenversagen, Niereninsuffizienz, zerebralen Vasospasmen, zerebraler Ischämie, Subarachnoidalblutungen, Migräne, Asthma, Atherosklerose, endotoxischem Schock, Endotoxin-induziertem Organversagen, intravaskulärer Koagulation, Restenose nach Angioplastie und Cyclosporin-induziertem Nierenversagen, bzw. Hypertonie.

Die gute Wirkung der Verbindungen läßt sich in folgenden Versuchen zeigen:

### Rezeptorbindungsstudien

Für Bindungsstudien wurden klonierte humane ET_{A}-Rezeptor-exprimierende CHO-Zellen und Meerschweinchen-Kleinhirnmembranen mit > 60 % ET_{B}- im Vergleich zu ET_{A}-Rezeptoren eingesetzt.

### Membranpräparation

Die ET_{A}-Rezeptor-exprimierenden CHO-Zellen wurden in F₁₂-Medium mit 10 % fötalem Kälberserum, 1 % Glutamin, 100 E/ml Penicillin und 0,2 % Streptomycin (Gibco BRL, Gaithersburg, MD, USA) vermehrt. Nach 48 h wurden die Zellen mit PBS gewaschen und mit 0,05 % trypsinhaltiger PBS 5 min inkubiert. Danach wurde mit F₁₂-Medium neutralisiert und die Zellen durch Zentrifugation bei 300 x g gesammelt. Zur Lyse der Zellen wurde kurz das Pellet mit Lysispuffer (5 mM Tris-HCl, pH 7,4 mit 10 % Glycerin) gewaschen und danach in einer Konzentration von 10⁷-Zellen/ml Lysispuffer 30 min bei 4°C inkubiert. Die Membranen wurden bei 20.000 x g 10 min zentrifugiert und das Pellet in flüssigem Stickstoff gelagert.

Meerschweinchenkleinhirne wurden im Potter-Elvejhem-Homogenisator homogenisiert und durch differentielle Zentrifugation 10 min bei 1.000 x g und wiederholte Zentrifugation des Überstandes 10 min bei 20.000 x g gewonnen.

### Bindungstests

Für den ET_{A}- und ET_{B}-Rezeptorbindungstest wurden die Membranen in Inkubationspuffer (50 mM Tris-HCl, pH 7,4 mit 5 mM MnCl₂, 40 µg/ml Bacitracin und 0,2 % BSA) in einer Konzentration von 50 µg Protein pro Testansatz suspendiert und bei 25°C mit 25 pM [125J]-ET₁ (ET_{A}-Rezeptortest) oder 25 pM [125J]-RZ₃ (ET_{B}-Rezeptortest) in Anwesenheit und Abwesenheit von Testsubstanz inkubiert. Die unspezifische Bindung wurde mit 10⁻⁷ M ET₁ bestimmt. Nach 30 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 mit 0,2 % BSA gewaschen. Die auf den Filtern gesammelte Radioaktivität wurde mit einem Packard 2200 CA Flüssigkeitszintillationszähler quantifiziert.

Die Bestimmung der Kᵢ-Werte erfolgte über nichtlineare Regressionsanalyse mit dem Programm LIGAND.

In Tabelle A ist die in der Versuchsanordnung ermittelte Wirkung von Verbindungen der Formel I als Kᵢ-Wert [mol/l] angegeben.

**Tabelle A**

| Verbindung | Kᵢ [mol/l] | |
|---|---|---|
| | ET-A | ET-B |
| 4.42 | 2,5 · 10⁻⁷ | 3,0 · 10⁻⁶ |
| 4.58 | 1,6 · 10⁻⁷ | 4,7 · 10⁻⁶ |

### Funktionelles in vitro-Testsystem für die Suche nach Endothelinrezeptor (Subtyp A)-Antagonisten

Dieses Testsystem ist ein funktioneller, auf Zellen basierender Test für Endothelinrezeptoren. Bestimmte Zellen zeigen, wenn sie mit Endothelin 1 (ET1) stimuliert werden, einen Anstieg der intrazellulären Calciumkonzentration. Dieser Anstieg kann in intakten Zellen, die mit Calcium-sensitiven Farbstoffen beladen wurden, gemessen werden.

Aus Ratten isolierte 1-Fibroblasten, bei denen ein endogener Endothelinrezeptor vom A-Subtyp nachgewiesen wurde, wurden mit dem Fluoreszenzfarbstoff Fura 2-an wie folgt beladen: Nach Trypsinierung wurden die Zellen in Puffer A (120 mM NaCl, 5 mM KC1, 1,5 mM MgCl₂, 1 mM CaCl₂, 25 mM HEPES, 10 mM Glucose, pH 7,4) bis zu einer Dichte von 2 x 10⁶/ml resuspendiert und in 30 min bei 37°C im Dunkeln mit Fura 2-am (2 µM), Pluronics F-127 (0,04 %) und DMSO (0,2 %) inkubiert. Danach wurden die Zellen zweimal mit Puffer A gewaschen und zu 2 x 10⁶/ml resuspendiert.

Das Fluoreszenzsignal von 2 x 10⁵ Zellen pro ml bei Ex/Em 380/510 wurde bei 30°C kontinuierlich registriert. Zu den Zellen wurden die Testsubstanzen zugegeben und nach einer Inkubationszeit von 3 min mit ET1 wurde die maximale Änderung der Fluoreszenz bestimmt. Die Antwort der Zellen auf ET1 ohne vorherige Zugabe einer Testsubstanz diente als Kontrolle und wurde gleich 100 % gesetzt.

In Tabelle B ist die in der Versuchsanordnung ermittelte Wirkung von Verbindungen der Formel I als IC₅₀-Wert [mol/l] angegeben.

**Tabelle B**

| Verbindung | IC₅₀ [mol/l] |
|---|---|
| 4.42 | 7,4 · 10⁻⁷ |
| 4.58 | 1,0 · 10⁻⁶ |

### Testung der ET-Antagonisten in vivo

Männliche 250 - 300 g schwere SD-Ratten wurden mit Amobarbital narkotisiert, künstlich beatmet, vagotomisiert und despinalisiert. Die Arteria carotis und Vena jugularis wurden kathetisiert.

In Kontrolltieren führt die intravenöse Gabe von 1 µg/kg ET1 zu einem deutlichen Blutdruckanstieg, der über einen längeren Zeitraum anhält.

Den Testtieren wurde 5 min vor der ET1 Gabe die Testverbindungen i.v. injiziert (1 ml/kg). Zur Bestimmung der ET-antagonistischen Eigenschaften wurde der Blutdruckanstieg in den Testtieren mit dem in den Kontrolltieren verglichen.

### Endothelin-1 induzierter "sudden death" an Mäusen

Das Testprinzip besteht in der Hemmung des durch Endothelin verursachten plötzlichen Herztodes der Maus, der wahrscheinlich durch Verengung der Herzkranzgefäße bedingt ist, durch Vorbehandlung mit Endothelin-Rezeptorantagonisten. Nach intravenöser Injektion von 10 nmol/kg Endothelin im Volumen von 5 ml/kg Körpergewicht kommt es innerhalb weniger Minuten zum Tod der Tiere.

Die letale Endothelin-1 Dosis wird jeweils an einem kleinen Tierkollektiv überprüft. Wird die Prüfsubstanz intravenös appliziert, erfolgt meist 5 min danach die im Referenzkollektiv letale Endothelin-1 Injektion. Bei anderen Applikationsarten verlängern sich die Vorgabezeiten, gegebenenfalls bis zu mehreren Stunden.

Die Überlebensrate wird dokumentiert und effektive Dosen, die 50 % der Tiere 24 h oder länger gegen den Endothelin-Herztod schützen (ED 50) werden ermittelt.

### Funktioneller Gefäßtest für Endothelin-Rezeptorantagonisten

An Aortensegmenten des Kaninchens wird nach einer Vorspannung von 2 g und einer Relaxationszeit von 1 h in Krebs-Henseleitlösung bei 37°C und einem pH-Wert zwischen 7,3 und 7,4 zunächst eine K⁺-Kontraktur ausgelöst. Nach Auswaschen wird eine Endothelin-Dosiswirkungskurve bis zum Maximum erstellt.

Potentielle Endothelin-Antagonisten werden an anderen Präparaten des gleichen Gefäßes 15 min vor Beginn der Endothelin-Dosiswirkungskurve appliziert. Die Effekte des Endothelins werden in % der K⁺-Kontraktur berechnet. Bei wirksamen Endothelin-Antagonisten kommt es zur Rechtsverschiebung der Endothelin-Dosiswirkungskurve.

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen etwa 0,5 und 50 mg/kg Körpergewicht bei oraler Gabe und zwischen etwa 0,1 und 10 mg/kg Körpergewicht bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1991). Die so erhaltenen Applikationsformen enthalten den Wirkstoff normalerweise in einer Menge von 0,1 bis 90 Gew.-%.

### Synthesebeispiele

### Synthese von Verbindungen der allgemeinen Formel VI

### Beispiel 1

### 3-Methoxy-3-(3-methoxyphenyl)-2-hydroxybuttersäuremethylester

19,5 g (88 mmol) 3-(3-Methoxyphenyl)-2,3-epoxybuttersäuremethylester werden in 200 ml absolutem Methanol gelöst und mit 0,1 ml Bortrifluorid-Etherat versetzt. Man rührt 12 Stunden bei Raumtemperatur und destilliert das Lösungsmittel ab. Der Rückstand wird in Essigester aufgenommen, mit Natriumbicarbonat-Lösung und Wasser gewaschen und über Natriumsulfat getrocknet. Nach Abdestillieren des Lösungsmittels verbleiben 21,1 g eines schwach gelben Öls.

Ausbeute: 94 % (Diastereomerengemisch 1:1)

### Beispiel 2

### 3-Benzyloxy-3-phenyl-2-hydroxybuttersäuremethylester

9,6 g (50 mmol) 3-Phenyl-2,3-epoxybuttersäuremethylester werden in 150 ml Benzylalkohol gelöst und mit 0,5 ml konzentrierter Schwefelsäure versetzt. Man rührt 6 Stunden bei 50°C und läßt auf Raumtemperatur abkühlen. Nach Neutralisation mit Natriumbicarbonat-Lösung destilliert man den überschüssigen Benzylalkohol am Hochvakuum ab und reinigt den Rückstand durch Flash-Chromatographie an Kieselgel mit n-Hexan/Essigester 9:1. Nach Abdestillieren des Lösungsmittels verbleiben 6,5 g eines farblosen Öls.

Ausbeute: 43 % (Diastereomerengemisch 3:2)

Analog wurden alle in Tabelle 1 genannten Verbindungen hergestellt.

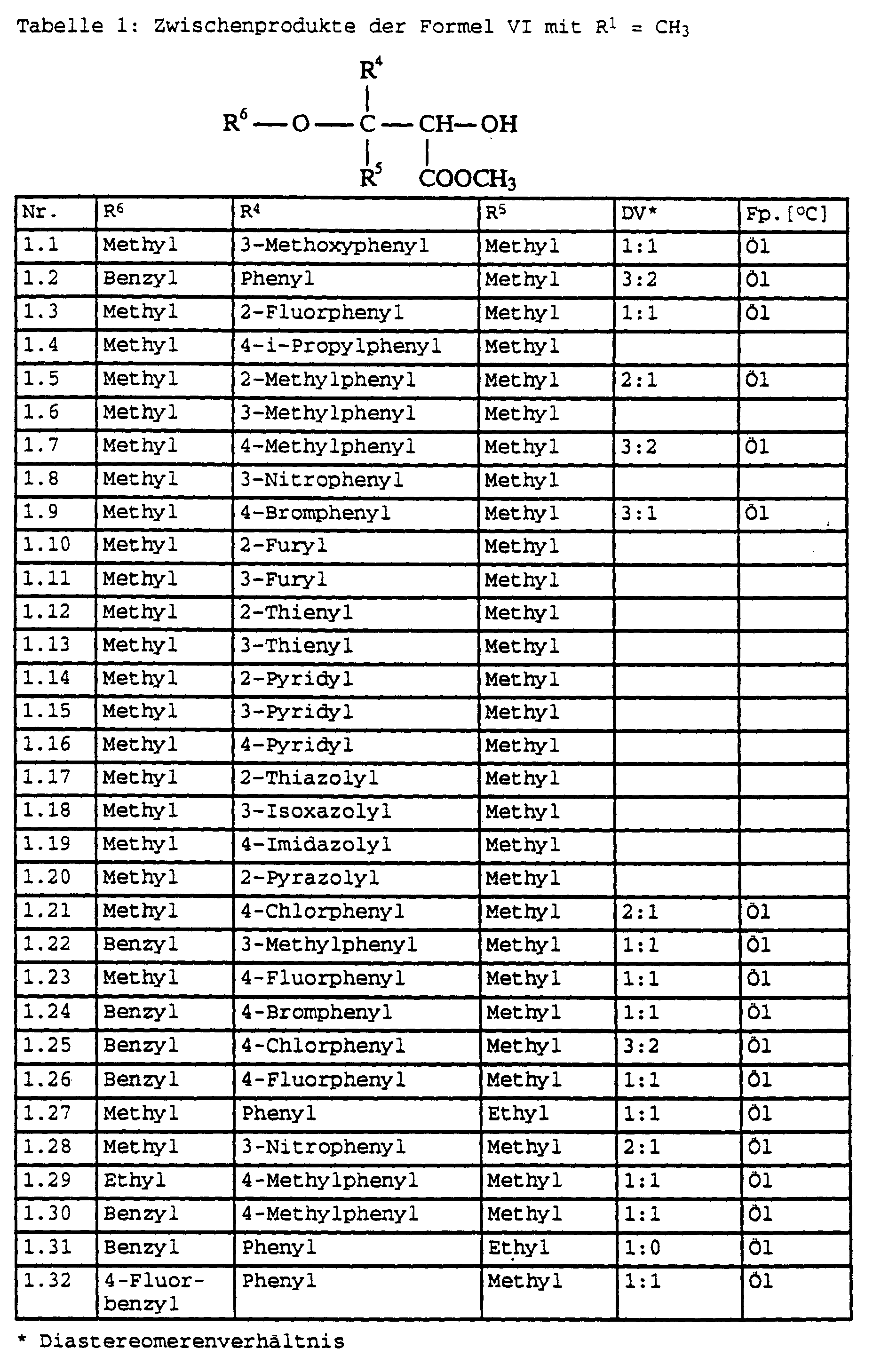

### Synthese von Verbindungen der allgemeinen Formel I:

### Beispiel 3:

### 3-Benzyloxy-3-phenyl-2-(4,6-dimethoxypyrimidin-2-yl)oxybuttersäure-methylester

3 g (10 mmol) 3-Benzyloxy-3-phenyl-2-hydroxybuttersäuremethylester (Verb. 1.2) werden in 40 ml Dimethylformamid gelöst und mit 0,3g (12mmol) Natriumhydrid versetzt. Man rührt 1 Stunde und gibt dann 2,2 g (10 mmol) 4,6-Dimethoxy-2-methylsulfonylpyrimidin zu. Nach 24 Stunden Rühren bei Raumtemperatur wird vorsichtig mit 10 ml Wasser hydrolisiert, mit Essigsäure ein pH-Wert von 5 eingestellt und das Lösungsmittel am Hochvakuum abdestilliert. Der Rückstand wird in 100 ml Essigester aufgenommen, mit Wasser gewaschen, über Natriumsulfat getrocknet und das Lösungsmittel abdestilliert. Der Rückstand wird mit 10 ml Methyl-t-butylether versetzt und der gebildete Niederschlag abgesaugt. Nach dem Trocknen verbleiben 2,4g eines weißen Pulvers.

Ausbeute: 55 % (Diastereomerengemisch 1:1) Fp.: 115 - 117°C

### Beispiel 4

### 3-Benzyloxy-3-phenyl-2-(4,6-dimethoxypyrimidin-2-yl)oxybuttersäure

1,4 g (3 mmol) 3-Benzyloxy-3-phenyl-2-(4,6-dimethoxypyrimidin-2-yl)-oxybuttersäuremethylester (Bsp. 3) werden in 20 ml Methanol und 20 ml Tetrahydrofuran gelöst und mit 3,7 g 10 % NaOH-Lösung versetzt. Man rührt 6 Stunden bei 60°C und 12 Stunden bei Raumtemperatur, destilliert die Lösungsmittel im Vakuum ab und nimmt den Rückstand in 100 ml Wasser auf. Nun wird mit Essigester zur Entfernung von nicht umgesetztem Ester extrahiert. Anschließend stellt man die Wasserphase mit verdünnter Salzsäure auf pH 1-2 und extrahiert mit Essigester. Nach Trocknen über Magnesiumsulfat und Abdestillieren des Lösungsmittels wird der Rückstand mit wenig Aceton versetzt und der gebildete Niederschlag abgesaugt. Nach dem Trocknen verbleiben 1,2 g eines weißen Pulvers.

Ausbeute: 88 % Diastereomerengemisch 3:2
Fp.: 165°C (Zersetzung)

### Beispiel 5

### 3-Benzyloxy-3-phenyl-2-[(4,6-dimethoxypyrimidin-2-yl)thio]-buttersäuremethylester

11 g (25 mmol) 3-Benzyloxy-3-phenyl-2-hydroxybuttersäuremethylester (Verb. 1.2) werden in 50 ml Dichlormethan gelöst, 3 g (30 mmol) Triethylamin zugegeben und unter Rühren 3,2 g (28 mmol) Methansulfonsäurechlorid zugetropft. Man rührt 2 Stunden bei Raumtemperatur, wäscht mit Wasser, trocknet über Magnesiumsulfat und engt im Vakuum ein. Der Rückstand wird in DMF aufgenommen und bei 0°C zu einer Suspension von 12,9 g (75 mmol) 4,6-Dimethoxypyrimidin-2-thiol und 8,4 g (100mmol) Natriumhydrogencarbonat in 100 ml DMF getropft. Nach 2 Stunden Rühren bei Raumtemperatur und weiteren 2 Stunden bei 60°C gießt man auf 1 1 Eiswasser und saugt den entstandenen Niederschlag ab. Nach Trocknen verbleiben 3,2 g eines weißen Pulvers.

Ausbeute: 29 % (Diastereomerengemisch 1:1)

Analog den obigen Beispielen wurden die in Tabelle 2 genannten Verbindungen hergestellt.

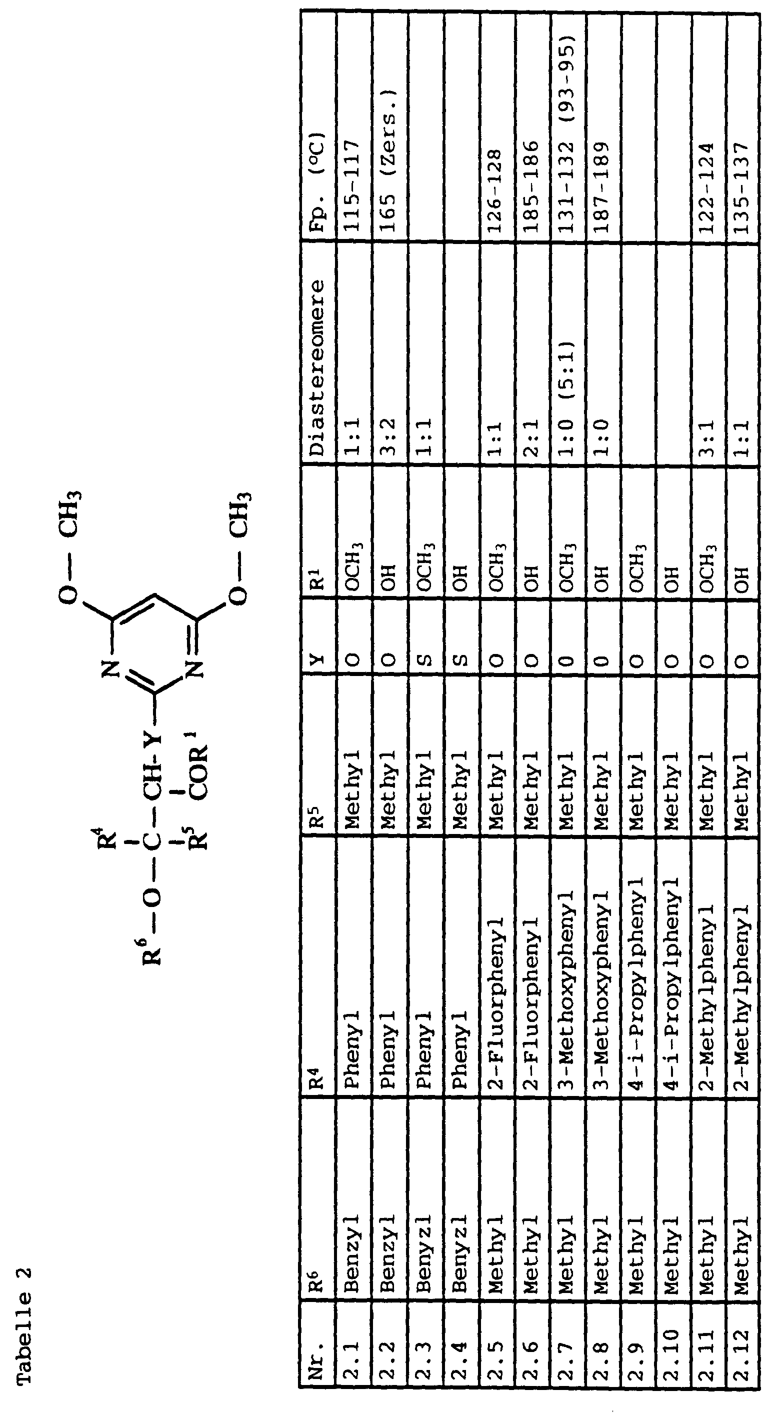

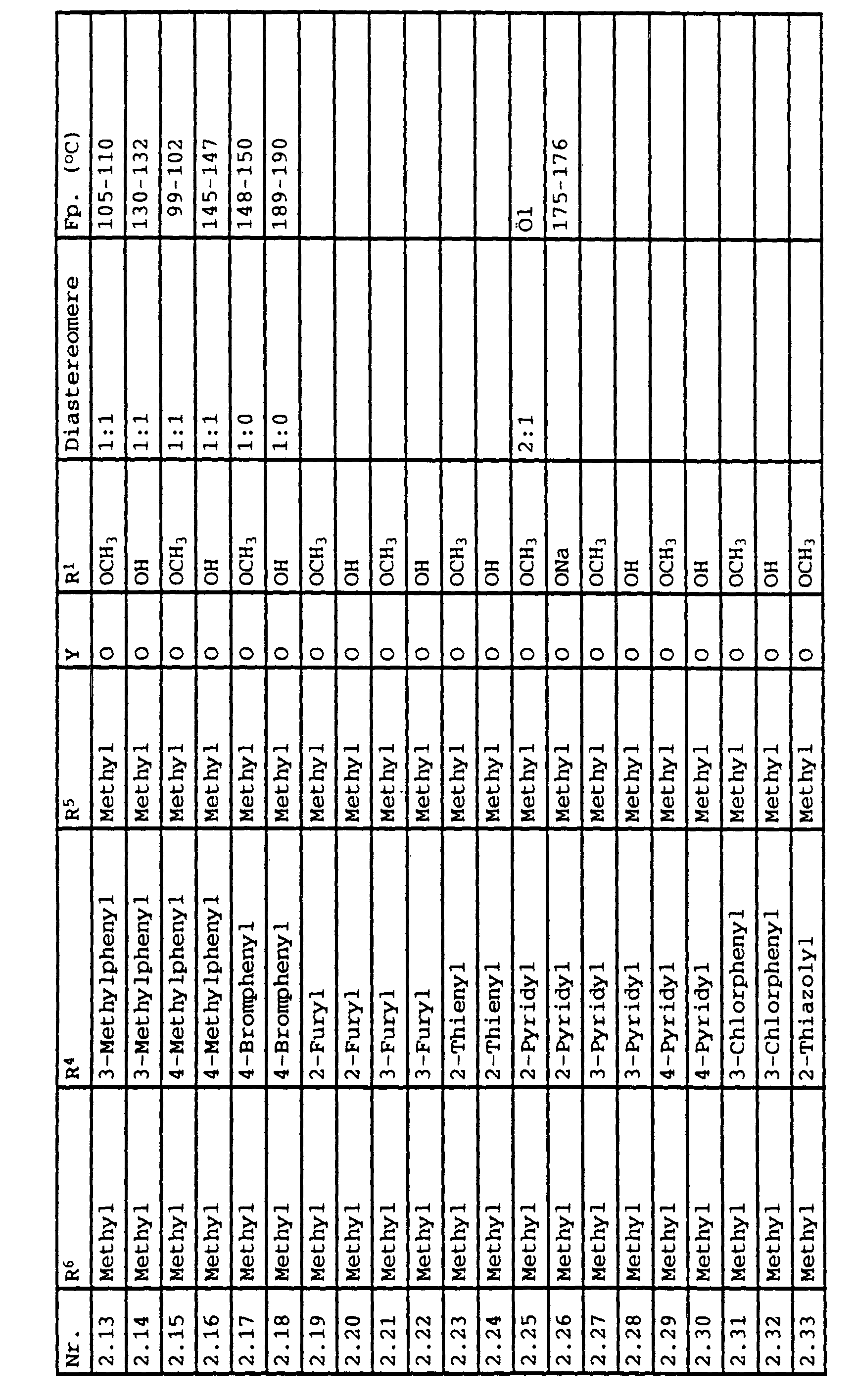

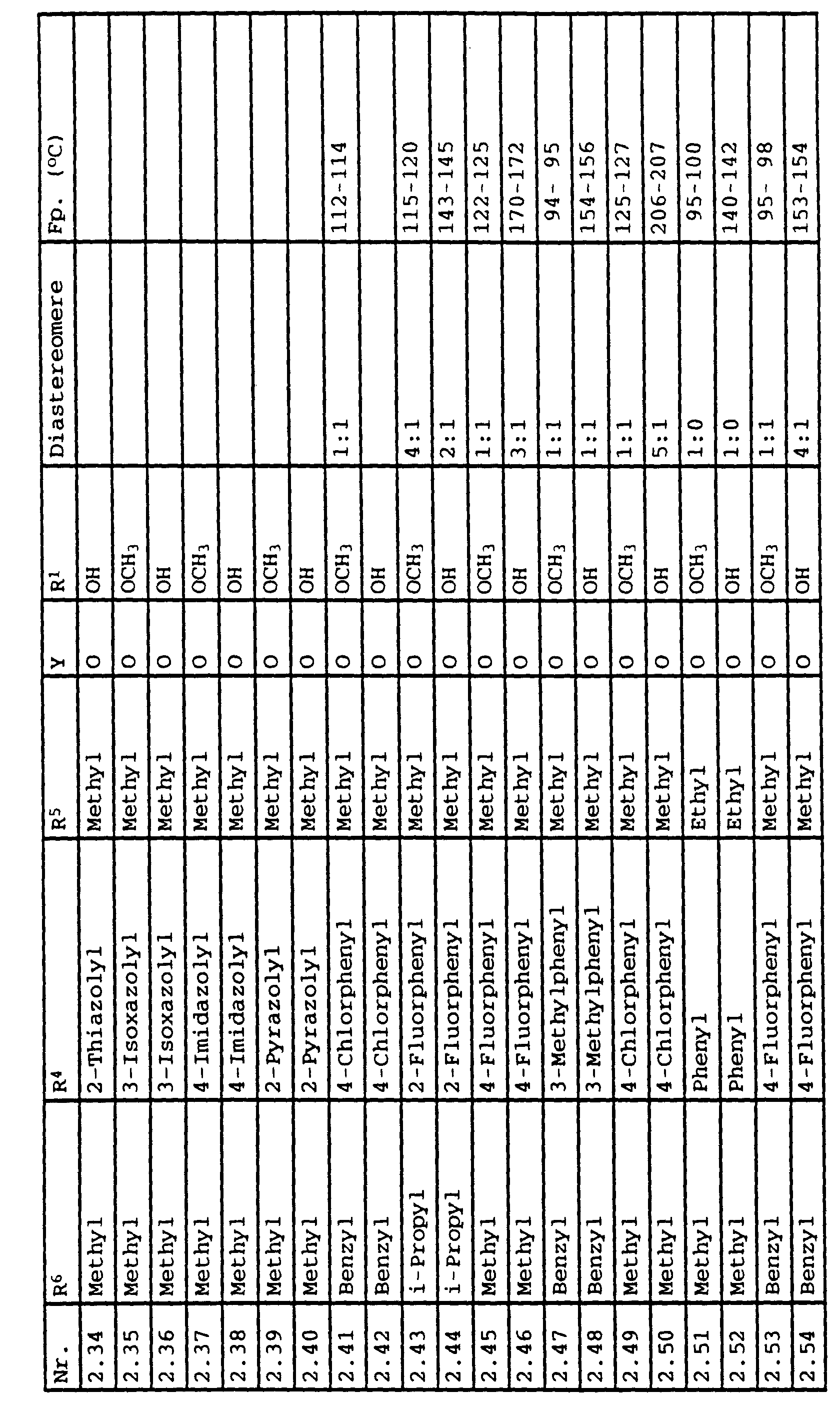

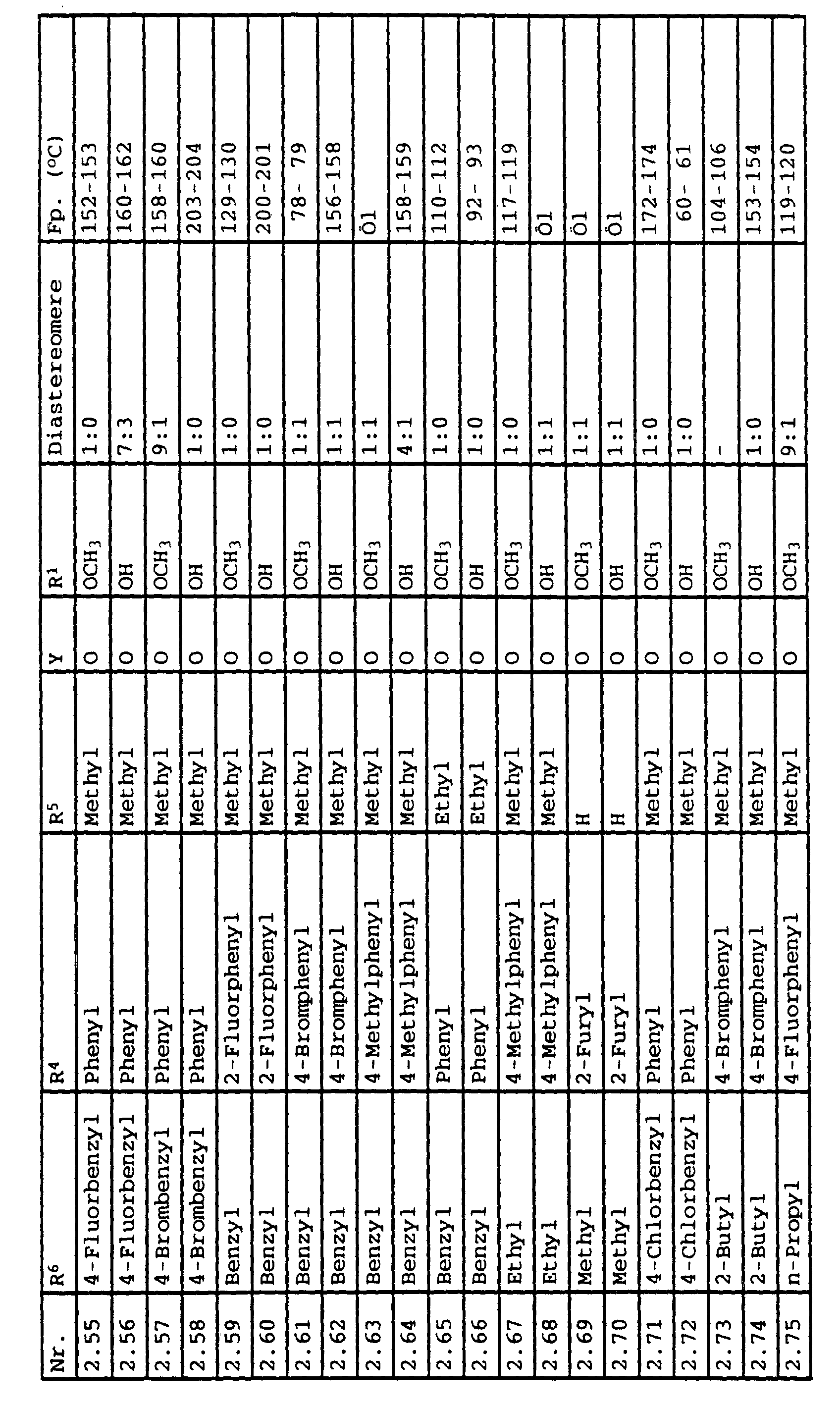

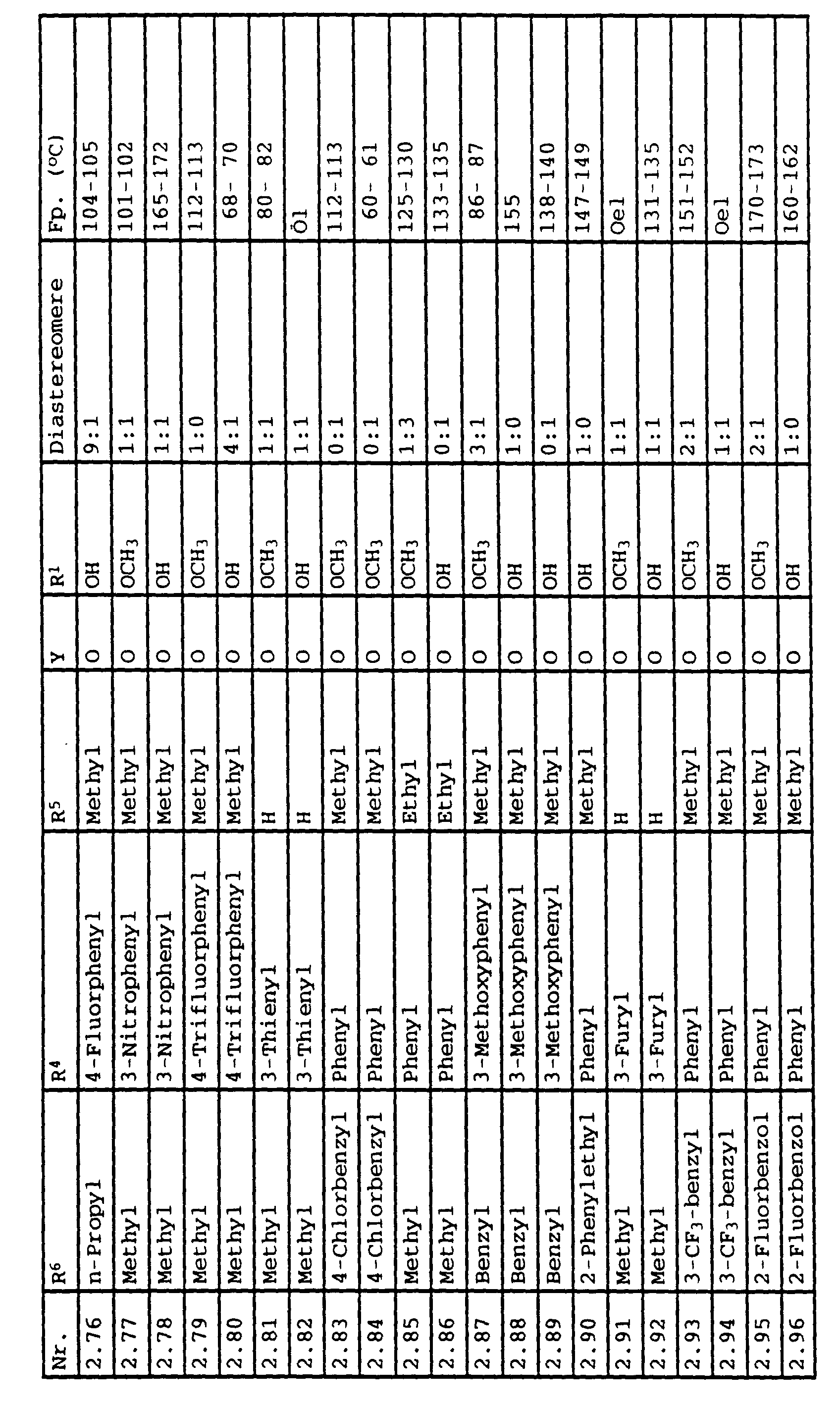

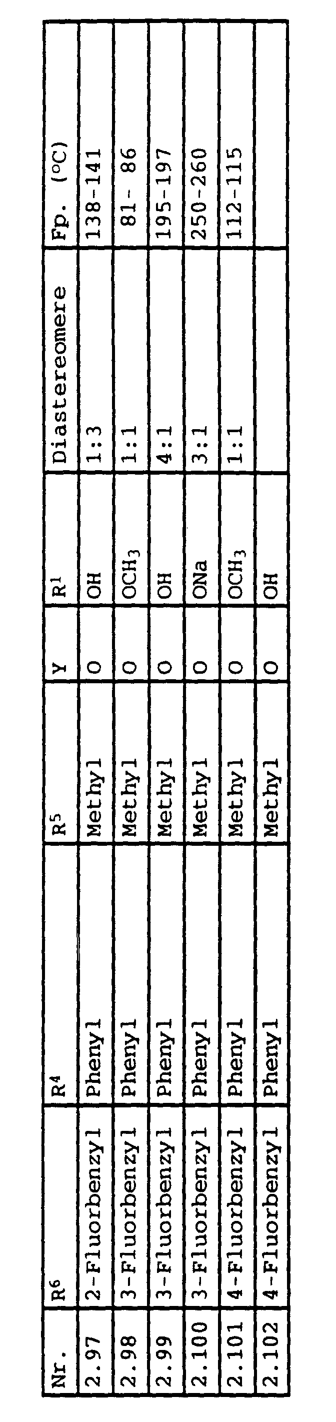

### Synthese von Verbindungen der allgemeinen Formel VI

### Beispiel 6

### 3-Phenoxy-3-phenyl-2-hydroxybuttersäuremethylester

28,2 g (0,3 mol) Phenol und 19,2 g (0,1 mol) 3-Phenyl-2,3-epoxybuttersäuremethylester werden zusammen 6 Stunden auf 100°C erhitzt. Nach Abdestillieren des überschüssigen Phenols am Hochvakuum und chromatographischer Reinigung des Rückstands an Kieselgel mit Hexan/Essigestergemischen erhält man 17,9 g eines schwach gelben Öls.

Ausbeute: 62,5 %

### Beispiel 7

### 3-(4-Bromphenyl)oxy-3-phenyl-2-hydroxybuttersäuremethylester

51,9 g (0,3 mol) 4-Bromphenol und 19,2 g (0,1 mol) 3-Phenyl-2,3-epoxybuttersäuremethylester werden 8 h bei 100°C und 12 h bei Raumtemperatur gerührt. Nach Abdestillieren des überschüssigen Phenols wird der Rückstand mittels Flash-Chromatographie (Kieselgel, n-Hexan-Essigester 9:1) gereinigt. Man erhält 7,2 g eines weißen Feststoffes.

Ausbeute: 20 %
Fp.: 133 - 135°C
Analog wurden die in Tabelle 3 genannten Verbindungen hergestellt:

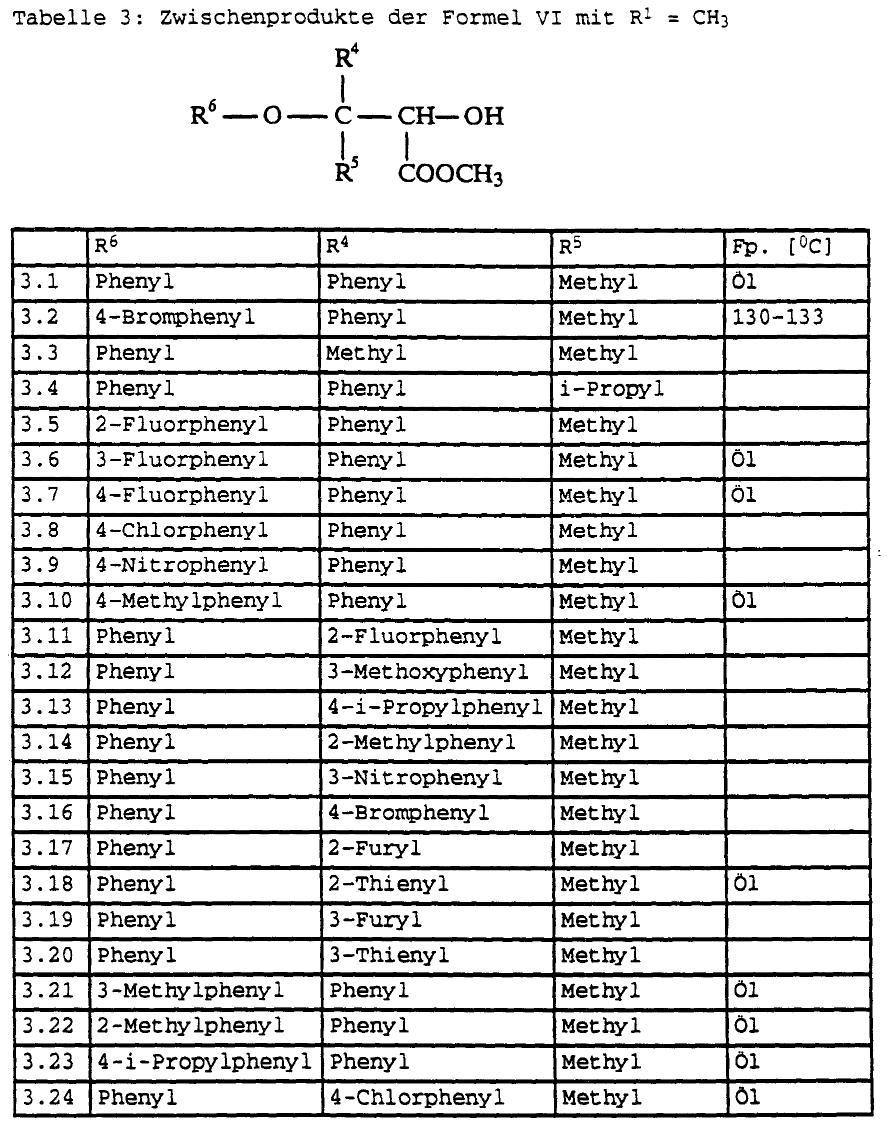

### Synthese von Verbindungen der allgemeinen Formel I:

### Beispiel 8

### 3-Phenoxy-3-phenyl-2-(4,6-dimethoxypyrimidin-2-yl)oxybuttersäure methylester

4,4 g (15,4 mmol) 3-Phenoxy-3-phenyl-2-hydroxybuttersäuremethylester (Verb. 1.1) werden in 40 ml Dimethylformamid gelöst und mit 0,46 g (18,4 mmol) Natriumhydrid versetzt. Man rührt 1 Stunde und gibt dann 3,4 g (15,4 mmol) 4,6-Dimethoxy-2-methylsulfonylpyrimidin zu. Nach 24 Stunden Rühren bei Raumtemperatur wird vorsichtig mit 10 ml Wasser hydrolisiert, mit Essigsäure ein pH-Wert von 5 eingestellt und das Lösungsmittel am Hochvakuum abdestilliert. Der Rückstand wird in 100 ml Essigester aufgenommen, mit Wasser gewaschen, über Natriumsulfat getrocknet und das Lösungsmittel abdestilliert. Der Rückstand wird mit 10 ml Methyl-t-butylether versetzt und der gebildete Niederschlag abgesaugt. Nach dem Trocknen verbleiben 1,6 g eines weißen Pulvers.

Ausbeute: 24,5 %
Fp.: 143 - 145°C

### Beispiel 9

### 3-Phenoxy-3-phenyl-2-(4,6-dimethoxypyrimidin-2-yl)oxybuttersäure

1,3 g 3-Phenoxy-3-phenyl-2-(4,6-dimethoxypyrimidin-2-yl)oxybuttersäuremethylester (Bsp. 8) werden in 20 ml MeOH und 40 ml Tetrahydrofuran gelöst und mit 3,7 g 10 % NaOH-Lösung versetzt. Man rührt 6 Stunden bei 60°C und 12 Stunden bei Raumtempertur, destilliert die Lösungsmittel im Vakuum ab und nimmt den Rückstand in 100 ml Wasser auf. Nicht umgesetzter Ester wird mit Essigester extrahiert. Anschließend stellt man die Wasserphase mit verdünnter Salzsäure auf pH 1 - 2 und extrahiert mit Essigester. Nach Trocknen über Magnesiumsulfat und Abdestillieren des Lösungsmittels verbleiben 1,0 g eines weißen Pulvers.

Ausbeute: 79,7 %
Fp.: 50 - 55°C

### Beispiel 10

### 3-Phenoxy-3-phenyl-2-[(4,6-dimethoxypyrimidin-2-yl)thio]buttersäuremethylester

7,2 g (25 mmol) 3-Phenoxy-3-phenyl-2-hydroxybuttersäuremethylester werden in 50 ml Dichlormethan gelöst, 3 g (30 mmol) Triethylamin zugegeben und unter Rühren 3,2 g (28 mmol) Methansulfonsäurechlorid zugetropft. Man rührt 2 Stunden bei Raumtemperatur, wäscht mit Wasser, trocknet über Magnesiumsulfat und engt im Vakuum ein. Der Rückstand wird in 100 ml DMF aufgenommen und bei 0°C zu einer Suspension von 12,9 g (75 mmol) 4,6-Dimethoxypyrimidin-2-thiol und 8,4 g (100 mmol) Natriumhydrogencarbonat in 100 ml DMF getropft. Nach 2 Stunden Rühren bei Raumtemperatur und weiteren 2 Stunden bei 60°C gießt man auf 1 Liter Eiswasser und saugt den entstandenen Niederschlag ab. Nach Trocknen verbleiben 4,2 g eines weißen Pulvers.

Ausbeute: 38 %

Analog den obigen Beispielen wurden die in Tabelle 4 genannten Verbindungen hergestellt.

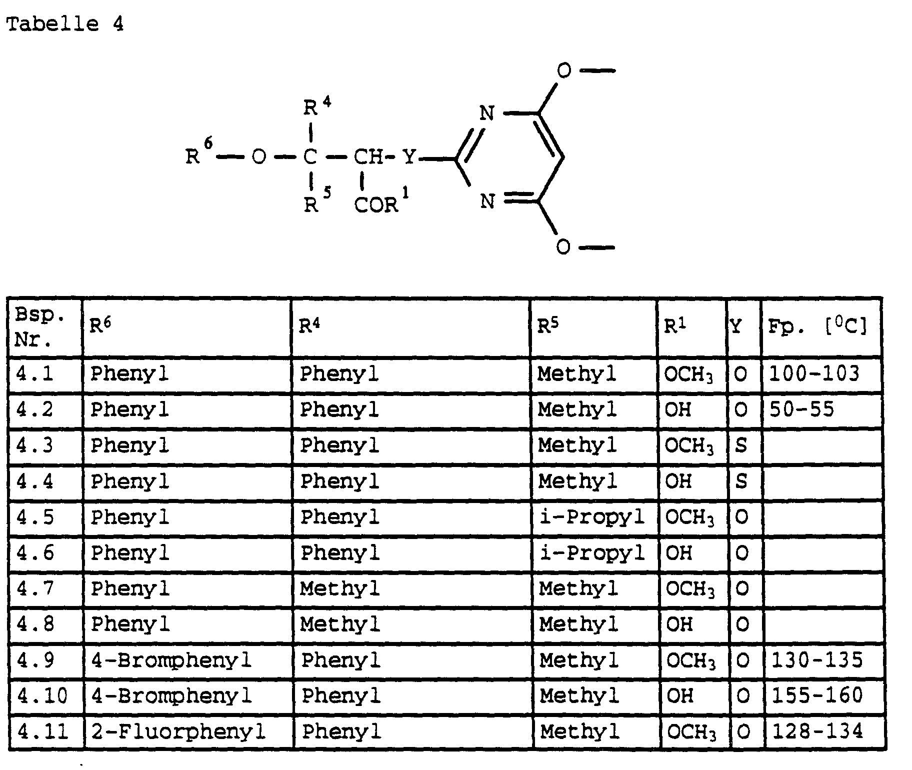

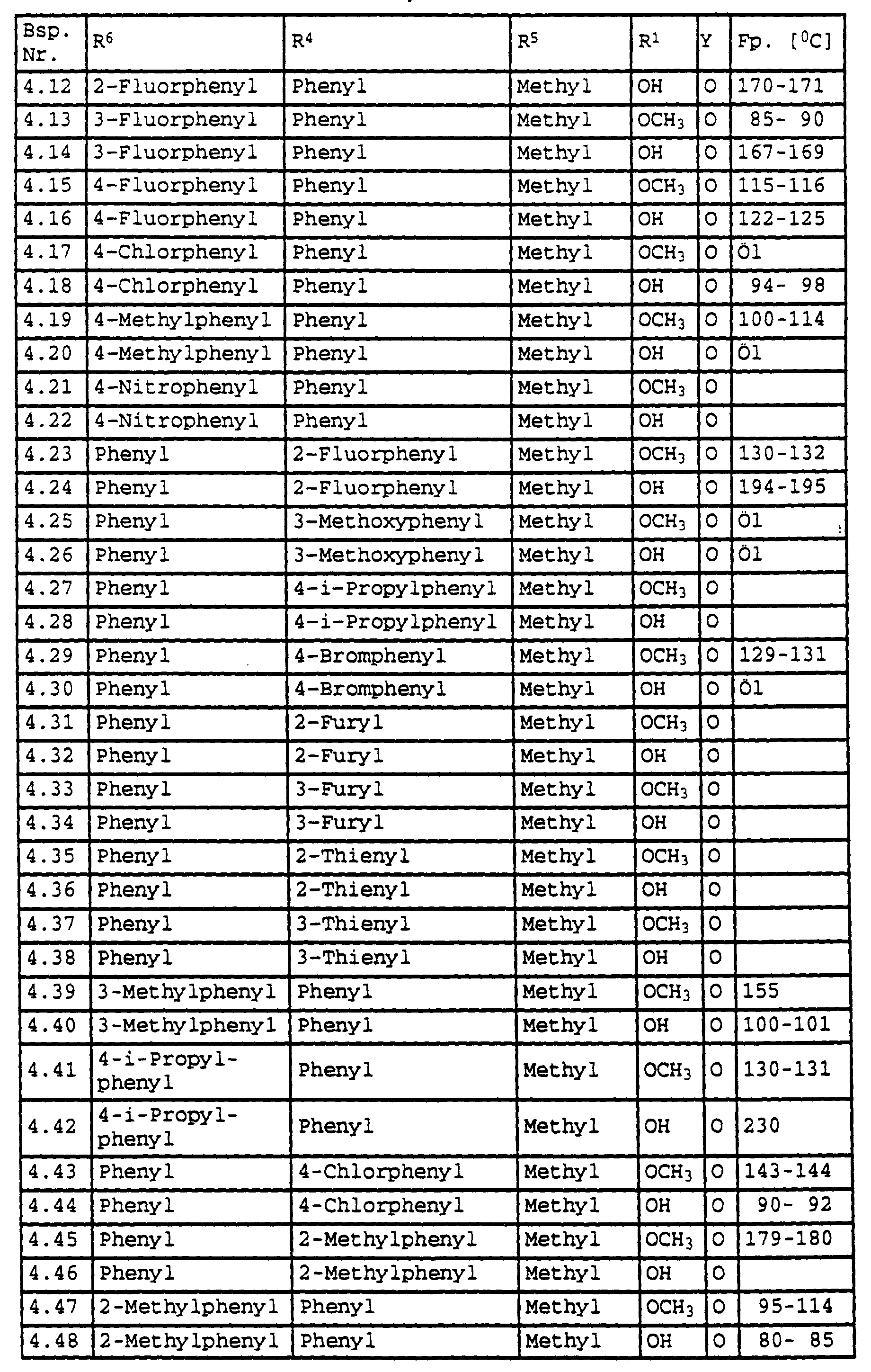

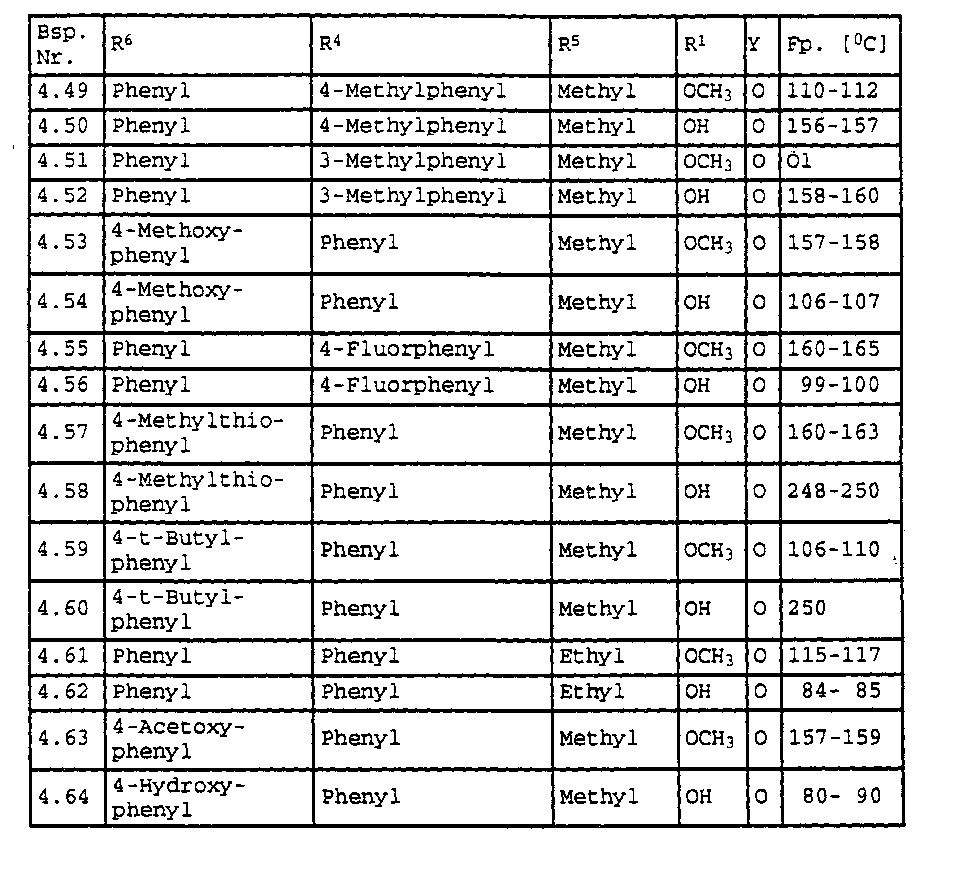

## Patentansprüche

1. Verwendung von Carbonsäurederivaten der Formel I 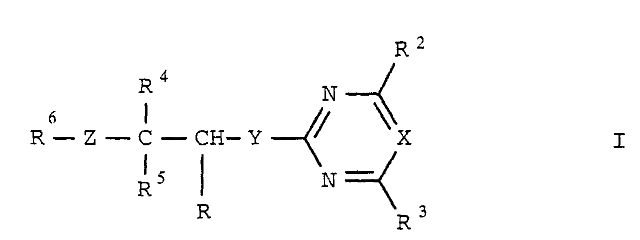 in der R eine Formylgruppe, eine Gruppe CO₂H oder einen zu COOH hydrolysierbaren Rest bedeutet und die übrigen Substituenten folgende Bedeutung haben:
R² Halogen, C₁-C₄-Alkyl, C₁-C₄-Halogenalkyl, C₁-C₄-Alkoxy, C₁-C₄-Halogenalkoxy oder C₁-C₄-Alkylthio;
X Stickstoff oder CR¹⁴, wobei R¹⁴ Wasserstoff bedeutet oder zusammen mit R³ eine 3- bis 4-gliedrige Alkylen- oder Alkenylenkette bildet, in der jeweils eine Methylengruppe durch Sauerstoff ersetzt ist;
R³ Halogen, C₁-C₄-Alkyl, C₁-C₄-Halogenalkyl, C₁-C₄-Alkoxy, C₁-C₄-Halogenalkoxy, C₁-C₄-Alkylthio oder R³ ist mit R¹⁴ wie oben angegeben zu einem 5- oder 6-gliedrigen Ring verknüpft;
R⁴ eine C₁-C₁₀-Alkylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₁-C₁₀-Alkylgruppe, welche ein bis fünf Halogenatome tragen kann und einen der folgenden Reste trägt: ein fünfgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio und/oder Phenyl;
eine C₃-C₁₂-Cycloalkyl- oder C₃-C₁₂-Cycloalkenylgruppe, die ein Sauerstoff- oder Schwefelatom enthalten kann und ein bis fünf Halogenatome und/oder einen der folgenden Reste tragen kann: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₃-C₆-Alkenyl- oder eine C₃-C₆-Alkinylgruppe, welche jeweils ein bis fünf Halogenatome und/oder einen der folgenden Reste tragen kann: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
ein fünf- oder sechsgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
Phenyl oder Naphthyl, die durch einen oder mehrere der folgenden Reste substituiert sein können: Halogen, Nitro, Cyano, Hydroxy, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, C₁-C₄-Alkylamino oder C₁-C₄-Dialkylamino;
R⁴ und R⁵ bilden zusammen mit dem benachbarten Kohlenstoffatom einen 3- bis 8-gliedrigen Ring, der ein Sauerstoff- oder Schwefelatom enthalten kann und einen bis drei der folgenden Reste tragen kann: C₁-C₄-Alkyl, Halogen, C₁-C₄-Halogenalkyl, C₁-C₄-Alkoxy, C₁-C₄-Halogenalkoxy und/oder C₁-C₄-Akylthio;
R⁵ Wasserstoff, C₁-C₄-Alkyl, C₃-C₆-Alkenyl, C₃-C₆-Alkinyl, C₃-C₈-Cycloalkyl, C₁-C₄-Halogenalkyl, C₁-C₄-Alkoxyalkyl, C₁-C₄-Alkylthioalkyl, Phenyl oder R⁵ ist mit R⁴ wie oben angegeben zu einem 3- bis 8-gliedrigen Ring verknüpft;
R⁶ C₁-C₈-Alkyl, C₃-C₆-Alkenyl, C₃-C₆-Alkinyl oder C₃-C₈-Cycloalkyl, wobei diese Reste jeweils ein- oder mehrfach substituiert sein können durch: Halogen, Nitro, Cyano, C₁-C₄-Alkoxy, C₃-C₆-Alkenyloxy, C₃-C₆-Alkinyloxy, C₁-C₄-Alkylthio, C₁-C₄-Halogenalkoxy, C₁-C₄-Alkylcarbonyl, C₁-C₄-Alkoxycarbonyl, C₁-C₄-Alkylamino, Di-C₁-C₄-alkylamino, Phenyl, ein- oder mehrfach, z.B. ein bis dreifach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl oder Phenoxy;
Phenyl oder Naphthyl, die jeweils durch einen oder mehrere der folgenden Reste substituiert sein können: Halogen, Nitro, Cyano, Hydroxy, Amino, C₁-C₄-Alkyl, C₁-C₄-Halogenalkyl, C₁-C₄-Alkoxy, C₁-C₄-Halogenalkoxy, Phenoxy, C₁-C₄-Alkylthio, C₁-C₄-Alkylamino oder C₁-C₄-Dialkylamino;
ein fünf- oder sechsgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
Y Schwefel oder Sauerstoff oder eine Einfachbindung;
z Schwefel oder Sauerstoff;
zur Herstellung von Arzneimitteln.

2. Verwendung nach Anspruch 1 zur Herstellung von Hemmstoffen für Endothelinrezeptoren.

3. Verwendung nach Anspruch 1 und 2 zur Behandlung von Hypertonie, Niereninsuffizienz und Restenose nach Angioplastie.

## Claims

1. The use of carboxylic acid derivatives of the formula I 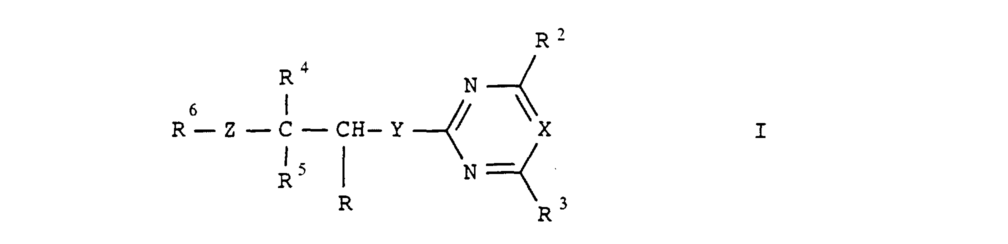 where R is formyl, CO₂H or a radical which can be hydrolyzed to COOH, and the remaining substituents have the following meanings:
R² is halogen, C₁-C₄-alkyl, C₁-C₄-haloalkyl, C₁-C₄-alkoxy, C₁-C₄-haloalkoxy or C₁-C₄-alkylthio;
X is nitrogen or CR¹⁴ where R¹⁴ is hydrogen or, together with R³, forms a 3- or 4-membered alkylene or alkenylene chain in which, in each case, one methylene group is replaced by oxygen;
R³ is halogen, C₁-C₄-alkyl, C₁-C₄-haloalkyl, C₁-C₄-alkoxy, C₁-C₄-haloalkoxy, C₁-C₄-alkylthio or R³ is linked to R¹⁴ as indicated above to form a 5- or 6-membered ring;
R⁴ is C₁-C₁₀-alkyl which can carry from one to five halogen atoms and/or one of the following radicals: C₁-C₄-alkoxy, C₁-C₄-alkylthio, cyano, C₁-C₈-alkylcarbonyl, C₁-C₈-alkoxycarbonyl, phenyl, phenoxy or phenylcarbonyl, where the phenyl radicals in turn can carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
C₁-C₁₀-alkyl which can carry from one to five halogen atoms and carries one of the following radicals: a five-membered heteroaromatic ring which contains from one to three nitrogen atoms and/or one sulfur or oxygen atom and which can carry from one to four halogen atoms and/or one or two of the following radicals: C₁-C₄-alkyl, C₁-C₄-haloalkyl, C₁-C₄-alkoxy, C₁-C₄-haloalkoxy, C₁-C₄-alkylthio and/or phenyl;
C₃-C₁₂-cycloalkyl or C₃-C₁₂-cycloalkenyl, each of which can contain one oxygen or sulfur atom and can carry from one to five halogen atoms and/or one of the following radicals: C₁-C₄-alkyl, C₁-C₄-alkoxy, C₁-C₄-alkylthio, cyano, C₁-C₈-alkylcarbonyl, C₁-C₈-alkoxycarbonyl, phenyl, phenoxy or phenylcarbonyl, where the phenyl radicals in turn can carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
C₃-C₆-alkenyl or C₃-C₆-alkynyl, each of which can carry from one to five halogen atoms and/or one of the following radicals: C₁-C₄-alkyl, C₁-C₄-alkoxy, C₁-C₄-alkylthio, cyano, C₁-C₈-alkylcarbonyl, C₁-C₈-alkoxycarbonyl, phenyl, phenoxy or phenylcarbonyl, where the phenyl radicals in turn can carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
a five- or six-membered heteroaromatic ring which contains from one to three nitrogen atoms and/or one sulfur or oxygen atom and which can carry from one to four halogen atoms and/or one or two of the following radicals: C₁-C₄-alkyl, C₁-C₄-haloalkyl, C₁-C₄-alkoxy, C₁-C₄-haloalkoxy, C₁-C₄-alkylthio, phenyl, phenoxy or phenylcarbonyl, where the phenyl radicals in turn can carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
phenyl or naphthyl, each of which can be substituted by one or more of the following radicals: halogen, nitro, cyano, hydroxyl, C₁-C₄-alkyl, C₁-C₄-haloalkyl, C₁-C₄-alkoxy, C₁-C₄-haloalkoxy, phenoxy, C₁-C₄-alkylthio, amino, C₁-C₄-alkylamino or C₁-C₄-dialkylamino;
R⁴ and R⁵ form, together with the adjacent carbon atom, a 3- to 8-membered ring which can contain one oxygen or sulfur atom and can carry from one to three of the following radicals: C₁-C₄-alkyl, halogen, C₁-C₄-haloalkyl, C₁-C₄-alkoxy, C₁-C₄-haloalkoxy and/or C₁-C₄-akylthio [sic];
R⁵ is hydrogen, C₁-C₄-alkyl, C₃-C₆-alkenyl, C₃-C₆-alkynyl, C₃-C₈-cycloalkyl, C₁-C₄-haloalkyl, C₁-C₄-alkoxyalkyl, C₁-C₄-alkylthioalkyl, phenyl or R⁵ is linked to R⁴ as indicated above to form a 3- to 8-membered ring;
R⁶ is C₁-C₈-alkyl, C₃-C₆-alkenyl, C₃-C₆-alkynyl or C₃-C₈-cycloalkyl, it being possible for each of these radicals to be substituted one or more times by: halogen, nitro, cyano, C₁-C₄-alkoxy, C₃-C₆-alkenyloxy, C₃-C₆-alkynyloxy, C₁-C₄-alkylthio, C₁-C₄-haloalkoxy, C₁-C₄-alkylcarbonyl, C₁-C₄-alkoxycarbonyl, C₁-C₄-alkylamino, di-C₁-C₄-alkylamino, phenyl, phenoxy or phenyl which is substituted one or more times, eg. from one to three times, by halogen, nitro, cyano, C₁-C₄-alkyl, C₁-C₄-haloalkyl, C₁-C₄-alkoxy, C₁-C₄-haloalkoxy or C₁-C₄-alkylthio;
phenyl or naphthyl, each of which can be substituted by one or more of the following radicals: halogen, nitro, cyano, hydroxyl, amino, C₁-C₄-alkyl, C₁-C₄-haloalkyl, C₁-C₄-alkoxy, C₁-C₄-haloalkoxy, phenoxy, C₁-C₄-alkylthio, C₁-C₄-alkylamino or C₁-C₄-dialkylamino;
a five- or six-membered heteroaromatic ring which contains from one to three nitrogen atoms and/or one sulfur or oxygen atom and which can carry from one to four halogen atoms and/or one or two of the following radicals: C₁-C₄-alkyl, C₁-C₄-haloalkyl, C₁-C₄-alkoxy, C₁-C₄-haloalkoxy, C₁-C₄-alkylthio, phenyl, phenoxy or phenylcarbonyl, where the phenyl radicals in turn can carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
Y is sulfur or oxygen or a single bond;
Z is sulfur or oxygen;
for the production of drugs.

2. The use as claimed in claim 1 for the production of inhibitors of endothelin receptors.

3. The use as claimed in claims 1 and 2 for the treatment of hypertension, renal insufficiency and restenosis after angioplasty.

## Revendications

1. Utilisation de dérivés d'acides carboxyliques de formule I 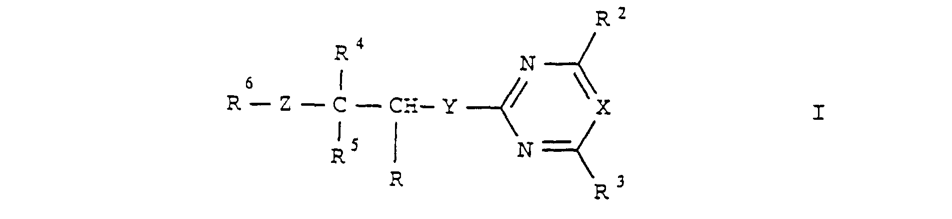 dans laquelle R représente un groupe formyle, un groupe CO₂H ou un groupe hydrolysable en groupe COOH et les autres symboles ont les significations suivantes :
R² : un halogène, un groupe alkyle en C1-C4, halogénoalkyle en C1-C4, alcoxy en C1-C4, halogénoalcoxy en C1-C4 ou alkylthio en C1-C4 ;
X : l'azote ou un groupe CR¹⁴ dans lequel R¹⁴ représente l'hydrogène ou forme avec R³ une chaîne alkylène ou alcénylène de trois à quatre chaînons et dans laquelle chacun groupe méthylène est remplacé par l'oxygène ;
R³ : un halogène, un groupe alkyle en C1-C4, halogénoalkyle en C1-C4, alcoxy en C1-C4, halogénoalcoxy en C1-C4, alkylthio en C1-C4, ou bien R³ forme avec R¹⁴, comme indiqué ci-dessus, un cycle à cinq ou six chaînons ;
R⁴ : un groupe alkyle en C1-C10 qui peut porter un à cinq atomes d'halogènes et/ou un des substituants suivants : alcoxy en C1-C4, alkylthio en C1-C4, cyano, (alkyle en C1-C8)carbonyle, (alcoxy en C1-C8)carbonyle, phényle, phénoxy ou phénylcarbonyle, les groupes phényle pouvant eux-mêmes porter un à cinq atomes d'halogènes et/ou un à trois des substituants suivants : alkyle en C1-C4, halogénoalkyle en C1-C4, alcoxy en C1-C4, halogénoalcoxy en C1-C4 et/ou alkylthio en C1-C4 ;
un groupe alkyle en C1-C10 qui peut porter un à cinq atomes d'halogènes et porte un des substituants suivants : un groupe hétéroaromatique à cinq chaînons contenant un à trois atomes d'azote et/ou un atome de soufre ou d'oxygène, qui peut porter un à quatre atomes d'halogènes et/ou un à deux des substituants suivants : alkyle en C1-C4, halogénoalkyle en C1-C4, alcoxy en C1-C4, halogénoalcoxy en C1-C4, alkylthio en C1-C4 et/ou phényle ;
un groupe cycloalkyle en C3-C12 ou cycloalcényle en C3-C12 qui peut contenir un atome d'oxygène ou de soufre et qui peut porter un à cinq atomes d'halogènes et/ou un des substituants suivants : alkyle en C1-C4, alcoxy en C1-C4, alkylthio en C1-C4, cyano, (alkyle en C1-C8)carbonyle, (alcoxy en C1-C8)carbonyle, phényle, phénoxy ou phénylcarbonyle, les groupes phényle pouvant eux-mêmes porter un à cinq atomes d'halogènes et/ou un à trois des substituants suivants : alkyle en C1-C4, halogénoalkyle en C1-C4, alcoxy en C1-C4, halogénoalcoxy en C1-C4 et/ou alkylthio en C1-C4 ;
un groupe alcényle en C3-C6 ou un groupe alcynyle en C3-C6, chacun d'eux pouvant porter un à cinq atomes d'halogènes et/ou un des substituants suivants : alkyle en C1-C4, alcoxy en C1-C4, alkylthio en C1-C4, cyano, (alkyle en C1-C8)carbonyle, (alcoxy en C1-C8)carbonyle, phényle, phénoxy ou phénylcarbonyle, les groupes phényle pouvant eux-mêmes porter un à cinq atomes d'halogènes et/ou un à trois des substituants suivants : alkyle en C1-C4, halogénoalkyle en C1-C4, alcoxy en C1-C4, halogénoalcoxy en C1-C4 et/ou alkylthio en C1-C4 ;
un groupe hétéroaromatique à cinq ou six chaînons contenant un à trois atomes d'azote et/ou un atome de soufre ou d'oxygène, qui peut porter un à quatre atomes d'halogènes et/ou un à deux des substituants suivants : alkyle en C1-C4, halogénoalkyle en C1-C4, alcoxy en C1-C4, halogénoalcoxy en C1-C4, alkylthio en C1-C4, phényle, phénoxy ou phénylcarbonyle, les groupes phényle pouvant eux-mêmes porter un à cinq atomes d'halogènes et/ou un à trois des substituants suivants :
alkyle en C1-C4, halogénoalkyle en C1-C4, alcoxy en C1-C4, halogénoalcoxy en C1-C4 et/ou alkylthio en C1-C4 ;
un groupe phényle ou naphtyle qui peut porter un ou plusieurs des substituants suivants : halogéno, nitro, cyano, hydroxy, alkyle en C1-C4, halogénoalkyle en C1-C4, alcoxy en C1-C4, halogénoalcoxy en C1-C4, phénoxy, alkylthio en C1-C4, amino, alkylamino en C1-C4 ou di-(alkyle en C1-C4)amino ;
R⁴ et R⁵ forment ensemble et avec l'atome de carbone voisin un cycle de trois à huit chaînons qui peut contenir un atome d'oxygène ou de soufre et peut porter un à trois des substituants suivants : alkyle en C1-C4, halogéno, halogénoalkyle en C1-C4, alcoxy en C1-C4, halogénoalcoxy en C1-C4 et/ou alkylthio en C1-C4 ;
R⁵ : l'hydrogène, un groupe alkyle en C1-C4, alcényle en C3-C6, alcynyle en C3-C6, cycloalkyle en C3-C8, halogénoalkyle en C1-C4, (alcoxy en C1-C4)alkyle, (alkyle en C1-C4)thioalkyle, phényle, ou bien R⁵ forme avec R⁴, comme indiqué ci-dessus, un cycle de trois à huit chaînons ;
R⁶ : un groupe alkyle en C1-C8, alcényle en C3-C6, alcynyle en C3-C6 ou cycloalkyle en C3-C8, chacun d'eux pouvant porter un ou plusieurs des substituants suivants : halogéno, nitro, cyano, alcoxy en C1-C4, alcényloxy en C3-C6, alcynyloxy en C3-C6, alkylthio en C1-C4, halogénoalcoxy en C1-C4, (alkyle en C1-C4)carbonyle, (alcoxy en C1-C4)carbonyle, alkylamino en C1-C4, di-(alkyle en C1-C4)amino, phényle, phénoxy ou phényle portant un ou plusieurs, par exemple un à trois substituants halogéno, nitro, cyano, alkyle en C1-C4, halogénoalkyle en C1-C4, alcoxy en C1-C4, halogénoalcoxy en C1-C4 ou alkylthio en C1-C4 ;
phényle ou naphtyle, chacun d'eux pouvant porter un ou plusieurs des substituants suivants : halogéno, nitro, cyano, hydroxy, amino, alkyle en C1-C4, halogénoalkyle en C1-C4, alcoxy en C1-C4, halogénoalcoxy en C1-C4, phénoxy, alkylthio en C1-C4, alkylamino en C1-C4 ou di-(alkyle en C1-C4)amino ;
un groupe hétéroaromatique à cinq ou six chaînons contenant un à trois atomes d'azote et/ou un atome de soufre ou d'oxygène, qui peut porter un à quatre atomes d'halogènes et/ou un à deux des substituants suivants : alkyle en C1-C4, halogénoalkyle en C1-C4, alcoxy en C1-C4, halogénoalcoxy en C1-C4, alkylthio en C1-C4, phényle, phénoxy ou phénylcarbonyle, les groupes phényle pouvant eux-mêmes porter un à cinq atomes d'halogènes et/ou un à trois des substituants suivants alkyle en C1-C4, halogénoalkyle en C1-C4, alcoxy en C1-C4, halogénoalcoxy en C1-C4 et/ou alkylthio en C1-C4 ;
Y : le soufre ou l'oxygène ou une liaison simple ;
Z : le soufre ou l'oxygène ;
pour la préparation de médicaments.

2. Utilisation selon la revendication 1, pour la préparation d'inhibiteurs des récepteurs de l'endothéline.

3. Utilisation selon les revendications 1 et 2, pour le traitement de l'hypertonie, de l'insuffisance rénale et d'un retour de sténose après angioplastie.
